# EUROPEAN PATENT SPECIFICATION

(11) **EP 1 513 502 B1**
(45) Date of publication and mention of the grant of the patent: **30.12.2015**
(21) Application number: 03702776.0
(22) Date of filing: 14.02.2003
(51) Int. Cl.: A61K 9/12, A61K 47/36, A61K 31/727, A61P 11/00

(54) **COMBINATION THERAPY FOR RESPIRATORY DISORDERS**
KOMBINATIONSTHERAPIE bei ATMUNGSSTÖRUNGEN
POLYTHERAPIE POUR LE TRAITEMENT DE TROUBLES RESPIRATOIRES

(30) Priority: 18.02.2002 GB 0203830; 18.02.2002 GB 0203773; 17.05.2002 GB 0211413; 18.10.2002 GB 0224328
(43) Date of publication of application: 16.03.2005
(73) Proprietor: Ockham Biotech Limited, Swanwick Hampshire SO31 7HA (GB)
(72) Inventor: SHUTE, Janis, Kay, Portsmouth Hampshire P06 2QP (GB); CARROLL, Mary Patricia Adult Cystic Fibrosis Unit, Southampton SO16 6YD (GB); CONWAY, Joy, Hampshire SO51 8DB (GB); HOCKEY, Peter, Morey, Lymington Hampshire SO41 5TB (GB)
(74) Representative: Woods, Geoffrey Corlett
(86) International application number: PCT/GB2003/000703
(87) International publication number: WO 2003/068188

(56) References cited:
- WO-A-02/32406
- GB-A- 1 560 463
- US-A- 5 633 003
- US-B1- 6 235 725
- DATABASE WPI Section Ch, Week 199330 Derwent Publications Ltd., London, GB; Class B04, AN 1993-239930 XP002240536 & JP 05 163161 A (DENKA SEIKEN KK), 29 June 1993 (1993-06-29)
- PATENT ABSTRACTS OF JAPAN vol. 1998, no. 04, 31 March 1998 (1998-03-31) & JP 09 309843 A (SHISEIDO CO LTD), 2 December 1997 (1997-12-02)
- SMITH A L ET AL: "Sputum changes associated with therapy for endobronchial exacerbation in cystic fibrosis.", THE JOURNAL OF PEDIATRICS APR 1988 LNKD- PUBMED:3127569, vol. 112, no. 4, April 1988 (1988-04), pages 547-554, ISSN: 0022-3476
- BALFOUR-LYNN I M ET AL: "Randomised controlled trial of inhaled corticosteroids (fluticasone propionate) in cystic fibrosis.", ARCHIVES OF DISEASE IN CHILDHOOD AUG 1997, vol. 77, no. 2, August 1997 (1997-08), pages 124-130, ISSN: 1468-2044
- J. Hirsh ET AL: "Mechanism of Action and Pharmacology of Unfractionated Heparin", Arteriosclerosis, thrombosis, and vascular biology, vol. 21, no. 7, 1 July 2001 (2001-07-01), pages 1094-1096, XP055088608, ISSN: 1079-5642, DOI: 10.1161/hq0701.093686

## Description

### Field of Invention

The present invention relates to the delivery of therapeutic agents to, and via, the lung and its airways.

### Background of Invention

Therapeutic agents are often administered directly to the respiratory tract via such methods as inhalation, instillation and intranasal delivery. This means that in respiratory disorders the therapeutic agent can be targeted directly to the site where it is needed. The respiratory route is often also used to deliver drugs to subjects with non-respiratory disorders and to healthy subjects. The respiratory route may also be use to administer non-therapeutic agents.

Administration of agents via the respiratory route has the advantage that it is typically quick and painless. Respiratory delivery also has the advantage that a therapeutic effect can be quickly achieved, whilst the potential for adverse effects is often lower than via other routes of administration. Furthermore, the subject can often deliver the agent themselves via this route and repeated doses can be given. Such delivery means are also relatively inexpensive.

A further advantage of pulmonary drug delivery is that following lung circulation, the majority of blood travels through the whole body before reaching the liver. Drugs administered orally are circulated much more quickly to the liver. This means that drugs administered via inhalation, intranasally or instillation have more opportunity to reach the whole of the body before their breakdown in the liver and hence can reach a wider range of targets at an effective concentration.

US 6,235,725 B1 is concerned with methods and compositions for the prevention of tolerance to medications.

### Summary of the Invention

The present invention is based on the finding that glycosaminoglycans can improve the delivery of an agent delivered via the inhaled, intranasal or instilled routes. The agent is a therapeutic agent. In particular, the invention is of use where the agent is being administered to a subject who is suffering from a condition characterised by elevated mucus viscosity and/or hypersecretion, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection. This unexpected effect means that less agent is necessary, i.e. has to be administered, to achieve the same effect in a given subject and also that effectively more agent can be delivered to the target site using, i.e. administering, the same amount of agent.

The higher level of bioavailability achievable with the same amount of agent administered may mean that conditions or individuals previously refractory to treatment with a particular therapeutic agent administered via inhalation, intranasal delivery or instillation, or which derived minimal benefit from such treatment, may now be treatable. It may also mean that less therapeutic agent has to be used for a given therapy and hence reduce the cost of the therapy.

The use of heparin as the glycosaminoglycan is especially preferred. Delivery via inhalation and/or intranasally is especially preferred.

Accordingly, the present invention provides for a glycosaminoglycan selected from chondroitin sulphates A to E, heparin, heparin sulphate, heparan, heparan sulphate and keratan sulphate or a physiologically acceptable salt of such a glycosaminoglycan for use in a method of treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is being treated with a therapeutic agent;
- the glycosaminoglycan or salt and the therapeutic agent are administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

The invention further provides a therapeutic agent for use in a method of treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is also administered a glycosaminoglycan selected from chondroitin sulphates A to E, heparin, heparin sulphate, heparan, heparan sulphate and keratan sulphate or a physiologically acceptable salt of such a glycosaminoglycan;
- the glycosaminoglycan or salt and the therapeutic agent are administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

In another instance, the invention further provides for the use of a glycosaminoglycan selected from chondroitin sulphates A to E, heparin, heparin sulphate, heparan, heparan sulphate and keratan sulphate or a physiologically acceptable salt of such a glycosaminoglycan in the manufacture of a medicament for treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is being treated with a therapeutic agent;
- the glycosaminoglycan or salt and the therapeutic agent are to be administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

The invention additionally provides for use of a therapeutic agent in the manufacture of a medicament for treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is also administered a glycosaminoglycan selected from chondroitin sulphates A to E, heparin, heparin sulphate, heparan, heparan sulphate and keratan sulphate or a physiologically acceptable salt of such a glycosaminoglycan;
- the glycosaminoglycan or salt and the therapeutic agent are to be administered via inhalation, intranasally and/or via instillation;
- the agent is to be delivered to the respiratory tract
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

The present invention further provides for products comprising a glycosaminoglycan selected from chondroitin sulphates A to E, heparin, heparin sulphate, heparan, heparan sulphate and keratan sulphate, or a physiologically salt of such a glycosaminoglycan and a therapeutic agent for simultaneous, separate or sequential use in the treatment of a human subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the glycosaminoglycan or salt and the therapeutic agent are to be administered via inhalation, intranasally and/or via instillation;
- the glycosaminoglycan or salt have an average molecular weight of from 12 to 18 kd;
- the agent is to be delivered to the respiratory tract;
- the mucus or the other pulmonary secretions contains extracellular genomic DNA which is compromising lung function; and
- the glycosaminoglycan increases delivery of the therapeutic agent.

Also referred to is a nebuliser or other liquid aerosol device, dry powder inhaler or metered dose inhaler comprising a pharmaceutical composition as described herein.

Also referred to is the use of a glycosaminoglycan, or a physiologically acceptable salt thereof, to increase the delivery of a non-therapeutic agent to, or via, the respiratory tract of a subject, where the glycosaminoglycan or physiologically acceptable salt thereof and the agent are administered to the subject via inhalation, instillation and/or intranasally.

### Brief Description of the Figures

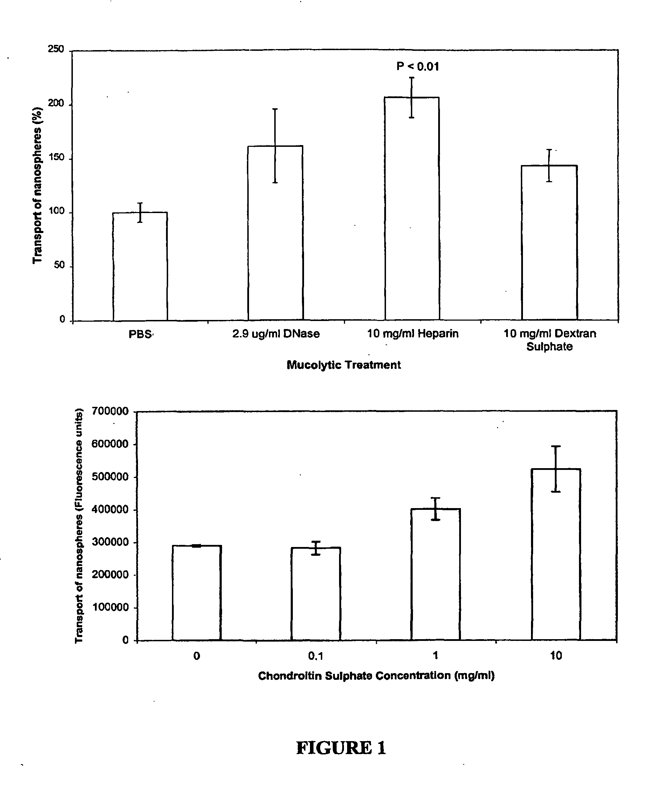

**Figure 1****: Effect of mucolytic agents on CAL/CF sputum (n=3).** As described in Example 1, CAL (chronic airflow limitation) or CF (cystic fibrosis) sputum was homogenised and mucolytic added on a 10 % v/w basis. Mixing was performed using a 19-gauge needle. Carboxylate-modified fluorescent beads were added to sputum sample in a 1:1 ratio (v/w). After vortexing, 20 µl of sample was added to each upper well of a micro-boyden chamber. The upper and lower wells of the chamber were separated by an 8 µm polycarbonate filter, and the lower wells contained 25 µl PBS. The chamber was centrifuged for five minutes at 1000 rpm to remove air bubbles prior to incubation at 37°C, 900 rpm, for four hours in the dark. Following incubation the chamber was dismantled and the fluorescence of the solution in the lower wells measured. The results for PBS, heparin, DNase and dextran sulphate (a non-glycosaminoglycan control) for CAL sputum are shown in the top panel of Figure 1. The results for a mixture of chondroitin sulphates A and C for CF sputum are shown in the bottom panel.

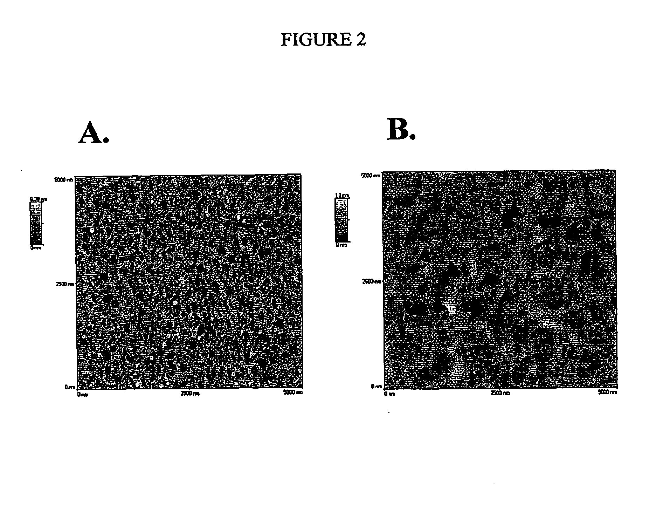

**Figure 2****: Atomic Force Microscopy (AFM) imaging of DNA.** As described in Example 2, calf thymus DNA (0.1 mg/ml) was treated with heparin (0.1 mg/ml) and AFM was performed in air under ambient conditions using a TopoMetrix TMX2000 Scanning Probe Microscope (ThermoMicroscopes, Bicester, UK). Panel A shows untreated DNA and Panel B shows DNA treated with heparin.

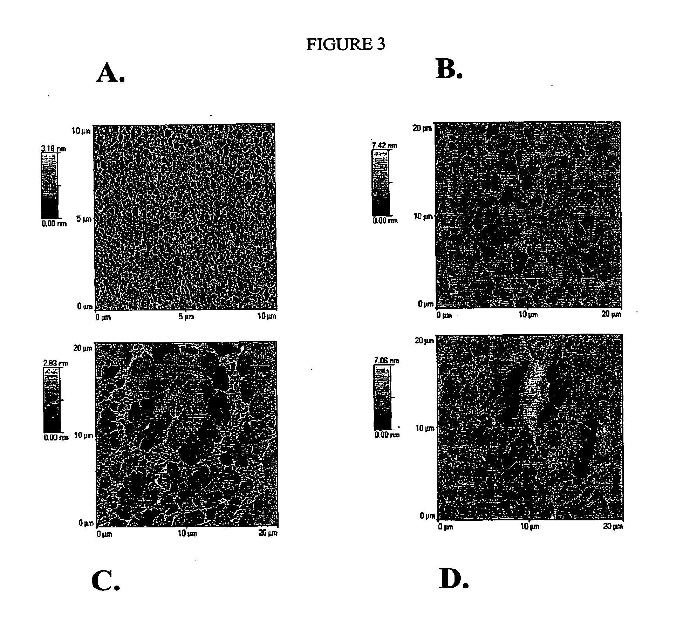

**Figure 3****: Atomic Force Microscopy (AFM) imaging of DNA.** Further Atomic Force Micrographs of DNA (100 µg/ml) treated with lower concentrations of heparin. Panel A shows the results for untreated DNA: Panel B for DNA treated with 0.1 µg/ml heparin; Panel C for DNA treated with 1 µg/ml heparin; and Panel D for DNA treated with 10 µg/ml heparin.

### Detailed Description of the Invention

The present invention is based on the finding that a glycosaminoglycan, or a physiologically acceptable salt thereof, can enhance the delivery of an agent to, or via, the respiratory tract. The glycosaminoglycan is selected from chondroitin sulphates A to E, heparin, heparin sulphate, heparan, heparan sulphate and keratan sulphate or is a physiologically acceptable salt of such a glycosaminoglycan. The glycosaminoglycan or salt thereof employed has an average molecular weight of from 12 to 18 kd. The activity of the agent is thus potentiated. The amount of agent delivered to the target site is therefore higher per unit of agent administered. A synergistic effect can be observed. The agent is a therapeutic agent.

The glycosaminoglycan or salt and the agent may be administered in the same or separate compositions and at the same time, simultaneously or sequentially.

### Synergy of glycosaminoglycan with therapeutic agents

Unexpectedly, glycosaminoglycans are capable of increasing the delivery of a therapeutic agent. That is that the same molar amount of agent administered results in a higher concentration of the agent being delivered to the target site when a glycosaminoglycan is also administered. In effect, the bioavailability of the agent at the target cells in the lung and/or its airways is increased. Glycosaminoglycans can also facilitate delivery intranasally via the nasal membranes. The glycosaminoglycan is thought to act by reducing the barrier function of the sputum, and other secretions of the lung and its airways, and hence allow the agent to reach the target cells in the airway more easily. The glycosaminoglycan is selected from chondroitin sulphates A to E, heparin, heparin sulphate, heparan, heparan sulphate and keratan sulphate or is a physiologically acceptable salt of such a glycosaminoglycan. The glycosaminoglycan or salt thereof employed has an average molecular weight of from 12 to 18 kd.

The amount of agent, and in particular therapeutic drug, passing through the mucus, or at the target cells, may be elevated by from one, two, three, ten, twenty or more fold by the addition of the glycosaminoglycan. The increase of the amount of the drug reaching the target cells, or in the mucus next to the target site, may typically be from 5% to 500%, preferably from 50 to 250%, and still more preferably from 50 to 100%.

The target for delivery of the drug may be any part of the respiratory tract including the nasal mucosal membranes. The agent may be administered via inhalation, instillation and/or intranasally and hence any part of the respiratory tract which these agents provide access to can be targeted. In some embodiments of the invention the target cells may be present in other parts of the body and the agent passes via the respiratory tract or nasal mucosal membranes to the blood and then onto the target cells. In such embodiments, any of the levels of increase or the amount of agent available, or present, specified herein may be seen in the blood stream and/or at, or in the vicinity of, the target cells. In some embodiments the aim may be to deliver the agent to the whole body or a specific organ, such as for example the brain:

The presence of the glycosaminoglycan may effectively increase bioavailability of the agent by more than one fold, double, treble, five fold, ten fold, twenty fold or more. Typically, the presence of the glycosaminoglycan may effectively reduce the molar amount, or amount by weight, of the agent necessary to achieve a given effect. The reduction may, for example, be by half, a quarter, a tenth, or more. It may reduce it by from 50 to 500%, preferably from 75 to 250%, more preferably by from 100 to 125%. Thus the amount of agent which has to be administered to achieve the same measurable effect may be decreased, for example, by any of these factors or by more than 10%, preferably more than 20%, more preferably more than 40%, even more preferably by more than 100%. The amount necessary to achieve the effect may be reduced by a factor of one, two, three, five, fifty, one hundred or more fold.

The amount of glycosaminoglycan added will typically be enough to cause a synergistic effect to increase the bioavailabilty of the agent. Thus the amount of glycosaminoglycan used may typically be the amount necessary to reduce the amount of agent which has to be administered to achieve a given effect. The reduction may be by any of the factors discussed herein. In particular, the effect measured may be a therapeutic effect.

The ratio of the agent to glycosaminoglycan by weight, or alternatively by units, may, for example, be from 1:50,000 to 1000:1, preferably from 1:10,000 to 100:1, more preferably from 1:5,000 to 50:1, still more preferably from 1:1,000 to 25:1. The ratio may, for example, be from 1:500 to 1:20, preferably be from 1:150 to 1:5, more preferably be from 1:50 to 1:2 and even more preferably be from 1:10 to 1:1. The two may, for example, be in equal amounts or the agent may be present in two fold, five fold, ten fold or greater excess or alternatively the glycosaminoglycan may be present in similar excess.

For a particular agent methods will be known to assess its efficacy and the effect that it has on a subject. Such methods may be employed in the production and assessment of the medicaments of the invention. Such methods may be used to determine the reduction in the amount of agent which may be administered, whilst still achieving the same effect, when a glycosaminoglycan is employed.

The elevation in bioavailabilty achievable using the invention may be assessed by measuring one or more of the effects of a given agent. These results may be used to decide on the necessary reduction in dose of agent to be administered and/or the increase in delivery rate achievable. Such methods may be used to decide on the ratio of agent to glycosaminoglycan and the amounts of each which will be present in the medicaments or compositions of the invention. A number of *in vitro* methods may also be used to assess this, including the barrier method discussed herein. Various labelled compounds may be used to measure the increase in delivery of the agent seen, for example, a labelled version of a therapeutic agent, such as a fluorescent or radioactively labelled version may be employed.

The increase in delivery of the agent achievable may be measured in terms of the concentration of the agent reaching, or at, the target site or through measuring a given effect of the agent. Any effect of a particular agent may be measured and in particular any effect whose magnitude is in relation to the amount of agent reaching the target site. In some cases, the size of the effect measured may be proportional to the amount of agent reaching the target site. In other cases, the effect measured may be a qualitative, rather than a quantitative one and the amount of agent required to generate it in the presence or absence of a particular glycosaminoglycan may be measured and preferably titrated. Standard curves of the effect achieved against the amount of agent administered in the presence or absence of a specific concentration of glycosaminoglycan may be generated. In a preferred embodiment, the assessment carried out will involve determining whether an effect is achieved which will be of benefit in treating the subject. In particular, an effect equivalent to that achieved when the recommended dose of an agent is administered in the absence of a glycosaminoglycan will be the target.

The effect may be a physiological or pharmacological one. The effect may be in any of the parameters described herein. The effect measured may be an improvement in the condition of a patient, such as a reduction in one or more symptom(s) of a disorder that they are suffering from, including those of any of the disorders mentioned herein.

Any of the parameters mentioned herein may be assessed. In particular, parameters associated with lung function, such as FEV₁, may be assessed. Mucus viscosity may be assessed. FEV₁ is defined as the maximal forced volume which can be expired in one second starting from maximum inspiration (European Resp. Journal, 1993; 6: Suppl. 16 and Coates, Lung Function: Assessment and Applications In Medicine, 4th Edition, Oxford, Blackwell, 1969). It can be measured by standard techniques well known in the art i.e. by spirometry.

Mucus viscosity may be determined by any of several different methods known in the art, including a sputum compaction assay (see for example WO 94/10567), assays using a torsion pendulum (Janmey, J. Biochem. Biophys. Methods 22:41-53 1991) or other suitable rheological methodologies.

FVC and in particular the ratio of FEV₁/FVC may also be assessed. FVC (forced vital capacity) corresponds to the maximal volume of air forcibly exhaled from the point of maximal inhalation and can be measured using standard spirometry.

In other embodiments the presence of an immune response may be measured, where the agent administered is an immunogen. In cases where the agent is a therapeutic gene, transformation efficiency may be measured. The efficiency of gene expression. at the RNA and/or protein level may be measured.

### Therapeutic agents

The therapeutic agent for use in the invention may be any therapeutic agent suitable for delivery via inhalation, intranasally or via instillation as the invention involves such delivery routes. In particular, the agent may be one suitable for delivery by inhalation and/or intranasally.

The therapeutic agent will be an agent that is suitable for treating chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection. The agent may be being administered prophylactically to prevent a disease. The agent may be one designed to reduce, eliminate or prevent worsening of one of the symptoms of the disorder. It may be one designed to prevent the development of such a symptom.

The therapeutic agent may be, for example, a bronchodilator, steroid, antimicrobial agent, antiviral agent, decongestant, or gene therapy vector. Examples of possible bronchodilators include salbutamol, salmeterol, ipratropium bromide, terbutaline sulphate, eformoterol and xanoate. Preferred steroids include beclomethasone dipropionate (BDP), budesonide, and fluticasone. Preferred antimicrobials include antibiotics (for example colistin), antivirals (such as relenza) and antiretrovirals (such as the HAART - highly active antiretroviral therapy - group of drugs), or antifungals (for example fluconazole and intraconazole).

The antiviral agent may be one effective against a virus with a double stranded or single stranded genome, such as a DNA or RNA virus. It may be an antiviral agent effective against any pathogenic virus, but in particular against one affecting the respiratory tract, or at least having part of its life cycle in the respiratory tract. It may be an ant-retroviral drug and in particular one against HIV (human immunodeficiency virus). The agent may be one against respiratory syncytial virus (RSV) such as, for example, ribavirin. The antiviral agent may be one against an influenza virus or a rhinovirus.

In the case of an antibacterial agent, the agent may be one against any pathogenic bacterium and in particular those affecting the respiratory tract. Examples of bacteria that the agent may be effective against include those responsible for pneumonia such as *Streptococcus pneumoniae, Mycoplasma pneumonie, Staphylcoccus aureus, Legionella pneumophila, Chlamydia pneumoniae* or *Chlamydia pstittaci.* The agent may be one effective against *Bordetella pertussis, Pseudomonas aeruginosa* or *Haemophillus influenzae.* It may be an agent effective against *Moraxella caterrhalis.* The agent may be active against more than one bacterium. For example, it may be active against two or more of any of the bacterium mentioned herein.

Gene delivery vectors for which the invention may be used to facilitate delivery include viral gene delivery vectors and, for example, gene delivery by adenovirus and adeno-associated virus. The vectors may include genes therapeutic for any of the conditions mentioned herein such as, for example, the CFTR gene or the α₁-antitrypsin gene. The methods of the invention may be used to increase the efficiency of other delivery methods. These include liposome, naked DNA, polycation complexes and peptide based delivery systems.

In cases where the agent is a liposome it may, for example, comprise a cationic lipid such as DOTAP (N-1(-(2,3-dioleoyloxy) propyl)-N,N,N-trimethyl ammonium methyl sulphate) or DOTMA (N-(1-(2,3-dioleoyloxyl) proply N,N,N-trimethyl ammonium chloride). The liposome may also comprise a neutral lipid, such as, DOPE (dioleoylphosphatidylethanolamine). In some embodiments of the invention the agent may be a liposome/polycation/nucleic acid complex. Such complexes may include polycations such as polylysine.

The agent may be a nucleic acid associated with a peptide to facilitate its delivery or packaging. For example, the agent may comprise the tetra-peptide sequence serine-proline-lysine-lysine, or repeats thereof, to facilitate condensation of the nucleic acid. The agent may comprise the peptide sequence tyrosine-lysine-alanine-(lysine)₃-tryptophan-lysine to facilitate condensation of the nucleic acid. The agent may comprise a nucleic acid-peptide complex associated with a molecule which results in specific binding, and preferably entry, into a particular cell type. For example, the complex may be conjugated with a ligand for a receptor and in particular one found on a cell type in the respiratory tract.

The therapeutic agent may be an ion channel modifier such as, for example, amiloride, UTP, ATP. Other possible secondary agents administered as the therapeutic agent include mucolytics, hormones, surfactants, proteins and peptides for systemic delivery, anti-anginals, vasopressin, analgaesic, or nicotine replacement therapy. In some embodiments of the invention, the therapeutic agent is, or will comprise, a bronchodilator, preferably a β₂ agonist, such as salmeterol and formoterol or an M₁ and/or M₃ antagonist, such as revatropate and triotropium bromide. The bronchodilator may be any of those used to treat asthma or chronic airflow limitation disorder (CAL).

In some embodiments of the invention, the therapeutic agent is a bronchodilator, preferably a β₂ agonist, such as salmeterol and formoterol, or an M₁ and M₃ antagonist, such as revatropate and triotropium bromide. The bronchodilator may be any of those used to treat asthma or chronic airflow limitation disorder. In further embodiments of the invention, the therapeutic agent may be an anti-inflammatory, preferably an LTD₄ antagonist, an LTB₄ antagonist, such as LY 293111, SC-53228, CP-105,696, SB 201146 and BIIL284, a 5'-lipoxygenase inhibitor, such as zileuton, a chemokine inhibitor, such as an interleukin 8 inhibitor or a MCP-1 anatgonist, a TNFα inhibitor, such as a monoclonal TNF antibody, a soluble TNF receptor and a TNF convertase inhibitor, an antioxidant, such as N-acetylcysteine, a stable glutathione analog and a nitrone, a prostanoid inhibitor, such as a COX-2 inhibitor, a thromboxane antagonist and an isoprostane receptor antagonist, theophylline, a phosphodiesterase-4-inhibitor, such as SB 207499, CP 80633 and CDP-840, a NF-kB inhibitor, an adhesion molecule inhibitor, such as a monoclonal antibody to CD18, ICAM-1 and E-selectin, interleukin 10, a p38 MAP kinase inhibitor, such as SB 203580, SB 220025 and RWJ 67657, colchicine or a selective EP₂ agonist, such as misoprostil and butaprost.

In yet further embodiments of the invention, the therapeutic agent is a protease inhibitor, preferably a neutrophil elastase inhibitor, such as ICI 200355, ONO-5046, MR-889 and L 658,758, a cathepsin inhibitor, such as suramin, a matrix metalloproteinase inhibitor, such as batimastat and marimastat, an α₁-antitrypsin, a serum protease inhibitor (serpin), such as elafin or a secretory leukoprotease inhibitor (SLPI). In further embodiments of the invention, the therapeutic agent is a mucoregulator, preferably a tachykinin agonist, such as CP-99,994 and SR 140333, a sensory neuropeptide release inhibitor, such as BW443 and levcromakalim, a cyclooxygenase inhibitor, such as indomethacin, a mucin (MUC) gene suppressor, such as a corticosteroid, a mucolytic agent or erythromycin.

In other embodiments of the invention, the therapeutic agent is an antibiotic, preferably a macrolide antibiotic, such as erythromycin, clarithromycin or roxithromycin. In further embodiments of the invention, the therapeutic agent is an antifungal agent, preferably amphotericin B or an azole, such as clotrimazole, ketoconazole, miconazole, flucouazole or itracouazole. In other embodiments of the invention, the therapeutic agent is a smoking cessator, preferably zyban.

The agent may be an immunogen designed to produce a therapeutic immune response, for example, the therapeutic agent may be a vaccine against a pathogen including any of those mentioned herein. The agent may, for example, comprise a protein, carbohydrate, and/or nucleic acid. The agent may comprise a small organic molecule. The agent may be an agonist and/or an antagonist and in particular may be an agonist, antagonist or modulator of a molecule which plays a role in any of the conditions mentioned herein. The agonistic or antagonistic effect of the agent may be responsible for the therapeutic effect of the agent. The agent may be an enzyme. In some embodiments the agent may be a prodrug which is activated following administration.

In cases where the therapeutic agent is a polypeptide it may be one which has been chemically modified to alter its properties such as, for example, its biological half-life. To achieve this covalent modifications may be introduced by reacting targeted amino acid residues of the native or variant polypeptide with an organic derivatising agent that is capable of reacting with selected amino acid side-chains or N- or C-terminal residues. Suitable derivatising agents and methods are well known in the art.

Residues which in particular may be derivatised include cysteinyl residues (most commonly by reaction with α-haloacetates), histidyl residues (by reaction with diethylpyrocarbonate at pH 5.5-7.0), lysinyl and amino terminal residues (by reaction with succinic or other carboxylic acid anhydrides), arginyl residues (by reaction with reagents such as phenylglyoxal, 2,3-butanedione, 1,2-cyclohexanedione, and ninhydrin). Carboxyl side groups (aspartyl or glutamyl) may be selectively modified by reaction with carbodiimides or may be converted to asparaginyl and glutaminyl residues by reaction with ammonium ions. The covalent attachment of agents such as polyethylene glycol (PEG) or human serum albumin to the therapeutic agents may reduce their immunogenicity and/or toxicity of the variant and/or prolong its half-life and hence may be used in the invention. In some embodiments of the invention the therapeutic agent may be directly conjugated to the glycosaminoglycan or joined through an intermediate molecule. It may be coated with a composition comprising the heparin.

In some embodiments of the invention more than one therapeutic agent may be used. For example, a glycosaminoglycan may be used to facilitate the delivery of two, three, four or more agents to the same subject. These may be present in the same or different compositions and be administered at the same time, in sequence or separately.

The target site which the therapeutic agent is to act on is preferably a cell type in the respiratory tract. However, it may be any, or all, cell types in the body. The therapeutic agent may be delivered from the lung to other sites via the blood system.

### Glycosaminoglycans

The medicaments and methods of the invention employ glycosaminoglycans. Glycosaminoglycans are linear heteropolysaccharides possessing characteristic disaccharide repeat sequences that are typically highly N-and O-sulpated at D-glucosamine, galactosamine and uronic acid residues. These sulphate moieties introduce a high degree of negative charge along the glycosaminoglycan polymer chain and add to the heterogeneity of these macromolecules.

The glycosaminoglycan is selected from chondroitin sulphates A to E, heparin, heparin sulphate, heparan, heparan sulphate and keratan sulphate or a physiologically acceptable salt of such a glycosoaminoglycan. The glycosaminoglycan or salt thereof has an average molecular weight of from 12 to 18 kd. The glycosaminoglycan will be in a form suitable for administration via inhalation, intranasally or via instillation as the medicaments of the invention will be delivered via such routes. Preferably, the glycosaminoglycan employed in the invention will be any of chondroitin sulphates A to E, heparin, heparin sulfate, heparan, heparan sulphate, keratan sulphate, or a mixture or any two thereof. Chondroitin sulphate B is sometimes referred to as dermatan sulphate. In a more preferred embodiment of the invention the glycosaminoglycan will be any of chondroitin sulphates A, C, D or E, heparin, heparin sulfate, heparan, heparan sulphate, keratan sulphate, or a mixture of any two thereof. In a particularly preferred embodiment the glycosaminoglycan will be chondroitin sulphate A, chondroitin sulphate C, heparin, heparin sulfate, heparan, heparan sulphate, or a mixture of any two thereof. More preferably, the glycosaminoglycan will be chondroitin sulphate A, chondroitin sulphate C, heparin, or a mixture of any two thereof. In an even more preferred embodiment of the invention the glycoaminoglycan will be heparin. In some embodiments of the invention the glycosaminoglycan employed will be a mixture of more than two glycosaminoglycans from one of the above mentioned groups, such as a mixture of three, four or five of the glycosaminoglycans.

In embodiments of the invention where a mixture of two glycosaminoglycans is employed the two may, for example, be present in the ratio 1:1, 1:2, 1:4, 1:10 or 1:100. The ratio maybe 90:10, 80:20, 70:30, or 60:40. Any suitable ratio may be employed and either glycosaminoglycan may be at the higher concentration. The ratio may be the same as the ratio in which the two are isolated when they are recovered from a common tissue using standard techniques. In a preferred embodiment of the invention a mixture of chondroitins A and C will be employed and in particular at a ratio of 80:20, preferably 75:25 and even more preferably 70:30 with chondroitin sulphate A being present at the higher level.

Typically, the glycosaminoglycan will not have been subjected to fragmentation to reduce its molecular weight. Usually, the glycosaminoglycan will not have been subjected to depolymerisation, such as by chemical or enzymatic means, to reduce its molecular weight. The average number of saccharide units in the polysaccharide chains of the glycosaminoglycan may, for example, be from 18 to 100, preferably from 30 to 80, more preferably from 40 to 60 and still more preferably from 5 to 60 units.

The glycosaminoglycan may be any suitable commercially available glycosaminoglycan and may, for example, be an unfractionated glycosaminoglycan. The glycosaminoglycan will have typically been isolated from a natural sources such as from an animal. In some cases, the glycosaminoglycan may have been synthesised rather than be a naturally occurring molecule.

In some cases the glycosaminoglycan may have been isolated from an animal, and in particular from animal tissues such as those of pigs or cattle. The glycosaminoglycan may have been obtained from tissues such as the lung, liver, or gut of an animal and in particular from beef lung or pork intestinal mucosa. The glycosaminoglycan may have been obtained from the skin of such an organism.

In some embodiments, the glycosaminoglycan may have been isolated from a cartilagenous fish or other sea or freshwater organism. In some cases the glycosaminoglycan may have been isolated from a shark or squid and in particular from the cartilage of such an organism. The glycosaminoglycan may have been isolated from a sturgeon and in particular from a sturgeon notochord.

Glycosaminoglycans and glycosaminoglycan salts suitable for use in the present invention will have an average molecular weight of from 12 to 18 kd. In particular, the glycosaminoglycan or salt may have an average molecular weight of from 14 to 18 kd, preferably from 15 to 17 kd and more preferably from 16 to 17 kd. In some embodiments all, or substantially all, of the glycosaminoglycan molecules or glycosaminoglycan salt molecules will have a molecular weight falling within the ranges specified above. Thus from 50 to 100%, preferably from 75 to 100%, more preferably from 90 to 100%, still more preferably 95 to 100% of the molecules may have such a molecular weight. In some cases at least 95%, preferably 97.5%, more preferably 99%, still more preferably 99.5% and even more preferably 99.9% may have a molecular weight falling within the range. The glycosaminoglycan or salt may be present in a range of molecular weight sizes and typically the most commonly occurring molecular weight size will fall within one of the above specified molecular weight ranges.

In the case of heparin, the heparin may have been subjected to O-desulphation such as at least at the 2-O and 3-O positions. The same or equivalent modifications may be made to other glycosaminoglycans.

The glycosaminoglycan may have been subjected to acetylation, deacetylation, oxidation and/or decarboxylation such as, for example, periodate oxidation. Heparinoids may be used in the invention.

The glycosaminoglycan employed is selected from chondroitin sulphates A to E, heparin, heparin sulphate, heparan, heparan sulphate and keratin sulphate or is a physiological salt of such a glycosaminoglycan, where the glycosaminoglycan or salt has an average molecular weight of from 12 to 18 kd.

Typically, the active compound used in the present invention comprises is a glycosaminoglycan or a physiologically acceptable salt thereof comprising repeating disaccharide units of general formula (1)

-[A-B]- (1)

wherein:
each A is the same or different and represents a moiety of formula (i) or (ii) 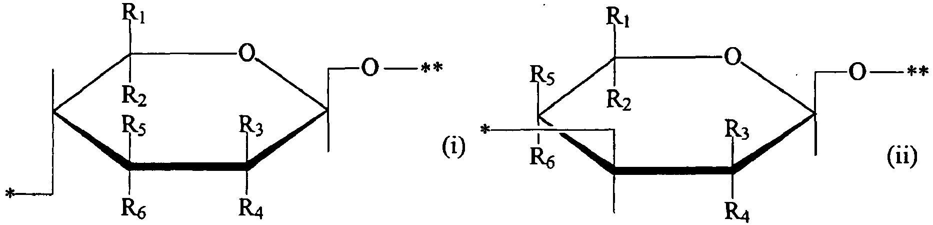
wherein:
- one of R₁ and R₂ is hydrogen, and the other is -CO₂H, -SO₃H or -CH₂OR wherein R is hydrogen or -SO₃H;
- one of R₃ and R₄ is hydrogen, and the other is -OR wherein R is hydrogen or -SO₃H;
- one of R₅ and R₆ is hydrogen, and the other is -OH;
- * represents a direct bond to an adjacent hydrogen atom or B moiety; and
- ** represents a direct bond to an adjacent B moiety;
each B is the same or different and represents a moiety of formula (iii) or (iv); 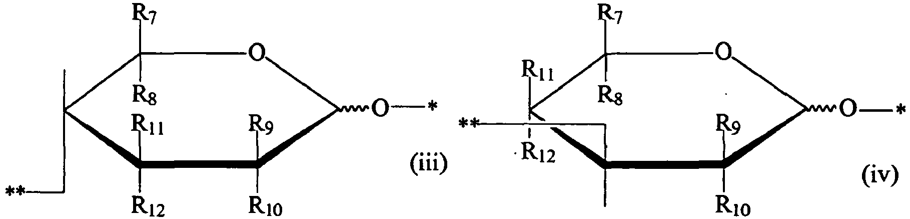 wherein:
- one of R₇ and R₈ is hydrogen and the other is -CH₂OH or -CH₂OSO₃H;
- one of R₉ and R₁₀ is hydrogen and the other is -NHAc, -NH₂ or -NHSO₃H;
- one of R₁₁ and R₁₂ is hydrogen and the other is -OH or -OSO₃H;
- * represents a direct bond to a hydrogen atom or an adjacent A moiety;
- ** represents a direct bond to an adjacent A moiety; and
- indicates a bond in either stereochemical orientation;
or a physiologically acceptable salt thereof.

The formulae herein adopt standard practice in depicting sugars. According to this practice, the formulae include vertical lines through each of the cyclic carbon atoms. This does not, of course, mean that methyl groups are attached at each position, or that methylene groups are present as part of the link between adjacent cyclic moieties.

Preferably, each A moiety in the glycosaminoglycan of general formula (1) is the same. Preferably, each B moiety in the glycosaminoglycan of general formula (1) is the same.

Preferably, each A moiety in the glycosaminoglycan of general formula (1) is a moiety of general formula (i).

Typically, one of R₁ and R₂ is hydrogen, and the other represents -CO₂H or -CH₂OR, wherein R is hydrogen or -SO₃H. Preferably, one of R₁ and R₂ is hydrogen and the other represents -CO₂H.

Typically, R₃ is hydrogen and R₄ is -OR, wherein R represents hydrogen or -SO₃H.

Typically R₅ is -OH and R₆ is hydrogen.

Typically each A is the same or different and represents a moiety of formula (i).

Typically R₇ is -CH₂OH or -CH₂OSO₃H and R₈ is hydrogen.

Typically R₉ is hydrogen and R₁₀ is -NHAc, -NH₂ or -NHSO₃H.

Typically R₁₁ is -OSO₃H or -OH and R₁₂ is hydrogen.

Typically each B is the same or different and represents 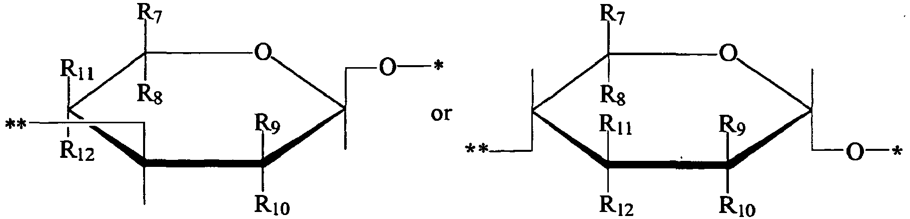 wherein R₇, R₈, R₉, R₁₀, R₁₁, R₁₂ * and ** are as described above.

Preferably R₁ is not hydrogen in an A moiety which is adjacent to a moiety (iv) in which R₁₂ is hydrogen.

Typically the glycosaminoglycan of general formula (1) is a glycosaminoglycan of general formula (2) 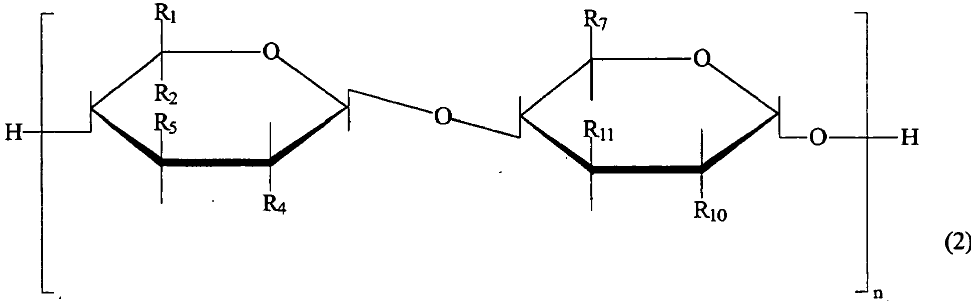 wherein:
- one of R₁ and R₂ is hydrogen and the other is -CO₂H;
- R₄ is -OH or -OSO₃H;
- R₅ is -OH;
- R₇ is -CH₂OH or -CH₂OSO₃H;
- R₁₀ is -NH₂, -NHSO₃H or -NHAc; and
- R₁₁ is -OSO₃H or -OH.

Typically the glycosaminoglycan of general formula (1) is a glycosaminoglycan of general formula (3) 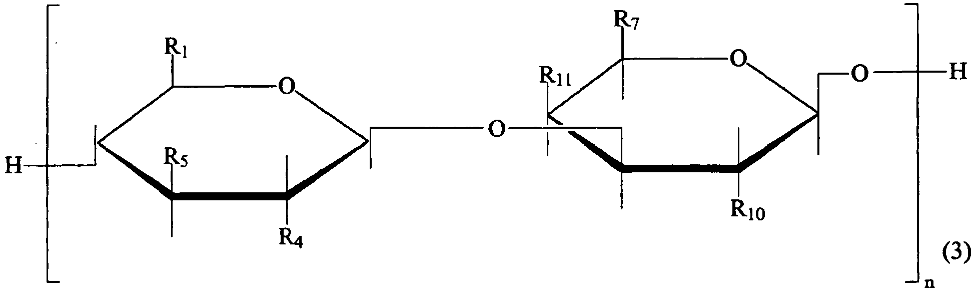 wherein:
- R₁ is -CO₂H;
- R₄ is -OH;
- R₅ is -OH;
- R₇ is -CH₂OH or -CH₂OSO₃H;
- R₁₀ is -NHAc; and
- R₁₁ is -OH or -OSO₃H.

Any suitable physiologically acceptable glycosaminoglycan salt may be employed in the invention and in particular a metallic salt such, for example a sodium salt, an alkali metal or an alkaline earth metal salt. Other salts include calcium, lithium and zinc salts. Ammonium salts may also be used. The salt may be a sodium glycosaminoglycanate or glycosaminoglycan sulphate. In the present application where mention of a glycosaminoglycan is made, such mention also includes physiologically acceptable salts thereof.

Typically a physiologically acceptable salt is a salt with a physiologically acceptable acid or base. Preferred salts are salts with physiologically acceptable bases. Typically, such salts are compounds wherein the acidic hydrogen atom of a -CO₂H and/or -OSO₃H group is replaced with a cation, for example an alkali metal (e.g. sodium or potassium) or alkali earth metal (e.g. calcium or magnesium) cation. Such salts can be prepared, for example, by reaction with an appropriate hydroxide.

The number of disaccharide units present in the glycosaminoglycan or salt thereof employed in the invention will be such that the average molecular weight of the glycosaminoglycan or salt is from 12 to 18 kd. In particular, it may be such that the glycosaminoglycan has a molecular weight of from 14 to 18 kd, preferably from 15 to 17 kd and more preferably from 16 to 17 kd. The number of disaccharide units present in the glycosaminoglycan may be represented by the number n, where n is any integer. Typically, n will be an integer of value such that the glycosaminoglycan has a molecular weight falling within any of the above mentioned molecular weight ranges.

Thus the glycosaminoglycan or salt employed may be represented by the general formula:

H-[A-B]ₙ-H

wherein -A-B is any of the disaccharides mentioned above and n is an integer. Typically n will be an integer of value such that the glycosaminoglycan or salt thereof has a molecular weight falling within the above specified molecular weight ranges. The value of n may be, for example, from 30 to 55 and more preferably from 35 to 50.

The glycosaminoglycan employed in the invention will typically comprise more than one length chain. Hence n for some of the glycosaminoglycan chains present may be a lower or higher integer than an integer which, on its own, would give a chain of molecular weight size falling within one of the above specified ranges. Thus the average value of n of the glycosaminoglycans present in the medicaments of the invention may be any of the values specified for n herein and in particular a value of n which gives a molecular weight glycosaminoglycan or salt falling within one of the molecular weight ranges specified herein. The average value of n for the glycosaminglycans in the medicaments of the invention may not necessarily be an integer due to a range of size chains being present, but in such cases will still fall within one of the above specified ranges.

Particularly preferred salts for use in the invention are salts of formula:

H-[A-B]ₙ^{x-}-H M^{x+}

wherein x≤4n and M represents a physiologically acceptable cation or a mixture thereof. Most preferably, x≤2n.

In a particularly preferred embodiment of the invention the glycosaminoglycan employed will be a heparin or a physiologically acceptable salt thereof. Heparin is a naturally occurring mucopolysaccharide present in a variety of organs and tissues, particularly liver, lung, and the large arteries. Heparin is a polymer of alternating α-D-glucosamine and hexuronate residues joined by (1,4) glycosidic linkages. When glycosaminoglycans are synthesised in nature, typically they are conjugated to a central protein core. However, preferably the glycosaminoglycans employed in the invention will lack such a central core. Typically, glycosaminoglycan preparations will lack a core and may be employed or, if present, the core can be removed. Commercially available preparations of glycosaminoglycans will usually lack the core and may be employed.

Heparin is clinically used as an anti-coagulant, where it is thought to exert its effects through interaction with anti-thrombin III (AT-III) and heparin co-factor II and other coagulation factors. Typically the heparin will retain some anticoagulant activity i.e. be able to increase clotting time in an individual. Thus preferably the heparin will be able to bind anti-thrombin III (AT-III) and/or heparin co-factor II (HCII) and hence inhibit clotting. Preferably it will be able to form a complex with AT-III, thrombin and a clotting factor. However, in some embodiments a heparin which lacks anti-coagulant activity or which has reduced anti-coagulant activity may also be employed. Thus the heparin may have been modified so that it has from 0 to 80%, preferably from 5 to 60%, more preferably from 10 to 40% and even more preferably from 10 to 30% of the activity of the unmodified form or in comparison to unmodified heparin. Other glycosaminoglycans, in particular dermatan sulphate, also possess anticoagulant activity. Preferably, therefore, the glycosaminoglycans employed will retain some anti-coagulant activity, as discussed above for heparin.

### Subjects

The subject is suffering from a respiratory disorder or disease selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection. The subject may be suffering from any disorder where delivery to, or via, the respiratory tract is appropriate for the therapeutic agent being administered. The subject to be treated will typically have a respiratory disease or a disorder which affects the respiratory system. The subject will suffer from, or be at risk of, a respiratory disorder where the viscosity of mucus, or of other pulmonary secretions, is elevated or increased. This increase may typically be by a factor of from one, two, three, four, five or more fold. It may be by more than 20%, more preferably by more than 50%, still more preferably by more than 80%. The increase is due to the presence of genomic DNA in the respiratory tract, such as that from necrotic inflammatory cells.

In cases where extracellular DNA is responsible for, or contributes to, an increase in mucus viscosity in the subject, it will typically be of high molecular weight. Thus it may be, for example, that it has an average fragment size, fragment size range or predominate fragment size in the range of from 100 bases to 1 megabase, preferably from 1 kb to 500 kb, more preferably from 5 kb to 250 kb in length, still more preferably from 25 kb to 100 kb and even more preferably from 25 kb to 50 kb in length. The DNA will typically be wholly, or partially free of, histones or will comprise regions which lack histones. It may be bound by cationic factors or factors with cationic groups such as inflammatory mediators and/or proteases. Thus for example it may be complexed or associated with elastase, cathepsins and/or IL-8. The presence of the DNA will typically increase the viscosity of the mucus, or other secretion, that it is present in.

Typically, the presence of DNA may increase the viscosity of the mucus by from 10 to 5000%, preferably from 50 to 2500%, more preferably from 100 to 1000%, still more preferably from 200 to 800% and even more preferably from 400 to 600%. The viscosity of the solution may typically be doubled, tripled, quadrupled or increased by five fold or more in comparison to the viscosity of the solution in the absence of the DNA. The viscosity of the DNA is such that it compromises lung function in the subject. It may cause airflow limitation and/or promote the occurrence of infections.

The increase in mucus viscosity or mucus levels may be due, or exacerbated by, the presence of factors which increase the secretion of mucus or other secretions, such as any of the irritants and pollutants discussed herein, and in particular tobacco smoke.

The respiratory disorder which the subject is suffering from may be, for example, an acute or chronic bronchial pulmonary disease, such as infectious pneumonia, bronchitis or tracheobronchitis, bronchiectasis, cystic fibrosis (CF), asthma, tuberculosis, and/or fungal infection. The subject may have a respiratory tract infection. The subject may have sinusitis, sinus congestion or viral infections which infect, or affect, the respiratory system such as a cold or flu. Hence, the subject has a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection. In a particularly preferred embodiment of the invention the subject will have cystic fibrosis, asthma or chronic airflow limitation disorder (CAL). The subject may have an infection of the respiratory tract. The subject may have pneumonia.

The disorder to be treated may involve infiltration of inflammatory cells to the lung and airways. These cells may be macrophages, granulocytes, lymphocytes or other white blood cells. They may be T cells or B cells. The granulocytes may be eosinophils, basophils or neutrophils and in particular will be neutrophils. The number of inflammatory cells present in the lung or airways, or at a particular site in the subject, may be increased by from 1 to 10,000 fold, preferably, from 10 to 5,000 fold, more preferably from 20 to 1,000 fold, still more preferably by from 50 to 500 fold and even more preferably from 100 to 200 fold. The proportion and number of given white cell types may be determined by standard staining techniques well known in the art.

Typically, many of the infiltrating inflammatory cells will be undergoing necrosis and/or lysis to release genomic DNA into the mucus of the individual. The lysis of such cells may also release other substances capable of increasing mucus viscosity. For example, actin, and in particular polymeric actin, may be present. The number and/or proportion of necrotic cells may be elevated typically by from 10 to 5000 fold, more preferably by from 50 to 2000 fold, still more preferably from 100 to 1000 fold and most preferably from 200 to 600 fold. In particular the necrotic infiltrating cells will be granulocytes and especially neutrophils and may be elevated by any of the levels mentioned herein. Their number may be increased by double, increased by ten, 100, 200 or 1000 fold.

The infiltrating inflammatory cells may be the source of genomic DNA responsible for the increase in mucus viscosity. The disorder involves the presence of genomic DNA which causes the increase in mucus viscosity, it may be predominately in the form of long strands of viscous genomic DNA. Proteins from the inflammatory cells such as inflammatory mediators and anti-bacterial proteins, such as proteases like neutrophil elastase and/or cathepsins, may also be present in the airways of the subject. As well as genomic DNA other polymers may also be present in the mucus further contributing to its viscosity.

A contributing factor to the migration of inflammatory cells to the lungs in the subject may be the presence of pathogens, irritants, allergens or pollutants in the lung. For example, and in particular in cystic fibrosis, the subject may have infections such as *Pseudomonas, Pneumococcus, Staphylococcus, Burkholderia*, *Haemophilus* and/or *Aspergillus* infections and in particular may display infection with *Haemophilus influenza, Staphylcoccus aureus, Pseudomonas aeruginosa,* and/or *Burkholderia cepacia.* These infections may be longstanding, occur periodically or be new. They may be acute or chronic. In some cases the pathogen will display antibiotic resistance due to prolonged exposure of antibiotics during treatment. In such cases the therapeutic agent will typically be an antibiotic which the pathogen is not resistant to.

Subjects may also, or alternatively, have a history of exposure to pollutants such as tobacco smoke or allergens such as pollen and these may contribute to the influx of inflammatory cells into the lung and/or the increase in mucus viscosity and levels. The presence of pathogen, allergens and/or pollutants may also promote the infiltrating cells to undergo necrosis, rather than be disposed of via processes such as apoptosis where genomic DNA is not believed to be released into the lung in substantial amounts and hence increase viscosity.

The subject may have a history of exposure to pollutants and/or chemicals and in particular to those in an inhaleable form. In particular, the subject may be a tobacco smoker or a former tobacco smoker. Typically, the subject may be, or have been, a heavy smoker, smoking from 10 to 100, preferably from 20 to 60, more preferably from 25 to 50 and even more preferably from 30 to 40 cigarettes per day. The subject may have done so for several years such as from 5 to 50, preferably from 10 to 40, more preferably from 15 to 30 and even more preferably from 20 to 25 years. The individual may have been, or be, a pipe or cigar smoker. The individual may have chewed tobacco or products containing tobacco. In some cases the individual may have been passively exposed to tobacco smoke rather than smoke tobacco themselves. Thus the subject may, for example, have been cumulatively exposed to passive tobacco smoke for long periods due to their work, leisure and/or home environments. The subject may use inhaled narcotic such as, for example, marijuana or other narcotics which are typically mixed with tobacco prior to smoking.

The subject may have additionally been, or alternatively be, exposed to other chemical or environmental pollutants. Thus the subject may work, or have worked, in an environment which exposes them to chemicals and/or pollutants. Thus, for example, the subject may be a factory worker or miner such as a coal miner. The subject may work in the construction industry. The subject may have been exposed to high levels of pollution, such as exhaust emissions from vehicles or other engines. The subject may have been exposed to smog or sulphur dioxide containing emissions. The subject may have a genetic predisposition to developing a disorder and in particular a respiratory disorder.

The subject may have a condition predisposing them to a disease which affects the respiratory system. For example, the subject may have α₁-antitrypsin deficiency. The subject may have cystic fibrosis and in particular possess one or two copies of the Δ508 mutation. The subject may have Chronic Airflow Limitation (CAL) disorder. The subject may have a mutated gene which affects mucus viscosity and/or the levels of mucus secretion or be infected with a pathogen which has such an effect

In one of the preferred embodiments of the invention the subject will have chronic airflow limitation (CAL). In one embodiment of the invention the subject may have chronic airflow limitation (CAL). CAL is a disease state characterized by airflow limitation that is not fully reversible. The airflow limitation is usually both progressive and associated with an abnormal inflammatory response of the lungs to noxious particles or gases. In particular, although not exclusively, CAL is associated with smoking.

For the purposes of the present invention CAL may be defined as a condition where there is a progressive decline in lung function, with the affected subject having an FEV₁ of less than 80% of that predicted for an individual of that age/race and/or height and/or who displays a FEV₁/FVC ratio of less than 70%. In an especially preferred embodiment, the subject to be treated with CAL will have an FEV₁ of less than 75% of that predicted. Typically the reduction of FEV₁ is only partially reversible. In particular the reduction in FEV₁ is only partially reversible by treatment with bronchodilators such as, for example, β2 adrenergic agonists and in particular salbutamol.

The FEV₁ for an individual with CAL may be from 10 to 80% of that predicted. Typically, the FEV₁ of the subject will be from 10 to 75% of the predicted value. Preferably the subject may have a FEV₁ of from 60 to 75% the predicted value, more preferably from 40 to 60% of predicted and even more preferably a value below 40% of that predicted. The subject with CAL may have an FEV₁ of less than 70%, preferably less than 60%, more preferably less than 50% and even more preferably less than 40% of that predicted. The FEV₁ value for the subject will normally be measured against predicted values and adjusted for age/sex/race and/or height. Predicted values may be those taken from Coates (*supra*). The expected value, which the value obtained for the subject with CAL may be compared to, may be the average expected value for smokers, or non-smokers, or both groups combined, preferably the expected value will be that for non-smokers not suffering from CAL (Coates, *supra*).

The reduction in FEV₁ in the subject with CAL will only be partially reversible and in particularly only be partially reversible on administration of a bronchodilator. Thus, for example, an increase in FEV₁ over the base-line value for the subject (i.e. that prior to administration of the bronchodilator) of more than 15%, preferably more than 20% and even more preferably over 25% will be regarded as reversibility. The increase may begin from 5 to 30, preferably from 10 to 25, more preferably over a period of from 15 to 20 minutes after the administration of the bronchodilator. Preferably the increases will begin from 15 minutes after the administration of the bronchodilator. The increases persist typically from 3 to 6 hours, preferably from 4 to 5 hours and more preferably 4 hours. Typically the bronchodilator used in assessing reversability will be a β₂ adrenergic agonist such as salbutamol, or ipratropium. In one embodiment of the invention the reduction in FEV₁ may be totally, or almost totally refractory to treatment with bronchodilators.

The subject with CAL may also show similar minimal increases in FEV₁ with steroid drugs such as Becotide™ or Prednisolone™ although typically response to such agents will not be used to define reversibility. The increases will also occur over a longer time period such as after 2 to 3 days and, if the steroid drugs are continually administered, persist. If steroids are stopped the improvement may persist for from 12 to 48 hours, or for days, weeks or even months, such as from six hours to six weeks, preferably from 1 day to 3 weeks.

Tests to assess reversibility of reduction of FEV₁ will typically be performed when the subject is clinically stable and free from infection. The subject should not have taken, or have had administered to them, inhaled short-acting bronchodilators in the previous six hours, long-acting β agonists in the previous 12 hours or sustained release theophyllines in the preceding 24 hours.

In the diagnosis of CAL spirometric values should typically be measured before and after an adequate dose of inhaled bronchodilator is given to the subject. The dose should preferably be selected to be high on the dose/response curve and usually will be given by nebuliser to be certain it has been inhaled. A similar dose may be given with multiple inhalations from a metered dose inhaler and large volume spacer, but this is less preferred. A typical dosage/measurements protocol for a human subject would be:
- before and 15 minutes after 2.5 to 5mg nebulised salbutamol or 5 to 10 mg terbutaline;
- before and 30 minutes after 500 :g nebulised ipratropium bromide; or
- before and 30 minutes after both in combination.

Equivalent protocols may be used for non-human subjects.

The FVC of the subject may also be measured in the diagnosis of CAL. The ratio of FEV₁ to FVC can be used in the diagnosis of CAL. Subjects to be treated who have CAL will typically have an FEV₁ /FVC value of less than 70%. The ratio of FEV₁ /FVC may be below 65%, preferably below 60%, more preferably below 55% and even more preferably below 55%. In an especially preferred embodiment, a subject will have a FEV₁ /FVC of below 70% and also have a FEV₁ value of 80% or less of that predicted.

FVC (forced vital capacity) corresponds to the maximal volume of air forcibly exhaled from the point of maximal inhalation and can be measured using standard spirometry. In particular, the above specified values for FEV₁ /FVC will be those after administration of a bronchodilator as outlined above. The reduction in FEV₁ /FVC will typically show the same lack of reversibility as FEV₁ in subjects with CAL.

Spirometric assessment is the most preferred method for diagnosing CAL and hence method for identification of subjects who may be treated. Accordingly, in an preferred embodiment of the invention spirometric assessment will be used in the diagnosis of a subject who has CAL and hence is treatable using the invention. In addition, the symptoms displayed by the subject may also be assessed to help confirm a diagnosis of CAL. Typically diagnosis will involve spirometric assessment in combination with assessment of the symptoms of a subject as well as elucidating whether the subject has a history of exposure to risk factors. In some situations spirometric assessment may not be possible, particularly in situations where resources are limited, and CAL will be diagnosed by alternative means such as by looking for the symptoms of CAL listed herein and a history of exposure to risk factors for CAL. Although chest X-rays are not typically indicative of whether or not a subject has CAL, they may be used to diagnose other respiratory disorders, such as TB, and hence rule out CAL.

The subject with CAL will show, or have previously shown, an accelerated rate of decline of lung function compared to the average expected for an equivalent individual not suffering from CAL and in particular for an equivalent non-smoking individual. The subject may display shortness of breath and in particular may do so after physical exertion such as on exercise. Typically, this will not be induced by exposure to an allergen. The subject may also show increased incidence of bacterial or viral infection and this may exacerbate the condition.

The individual with CAL may show a rate of decrease in FEV₁ one, two, three, four or more times greater than the average annual value expected for an equivalent individual not suffering from CAL. For instance a subject over thirty may show an annual reduction of from 50 to 100, preferably from 50 to 80 and more preferably from 60 to 70 ml FEV₁/yr compared to a reduction of from 10 to 40 and typically of 20 to 40 ml of FEV₁/yr in the equivalent non-smoker. These values may also apply to non smoking sufferers of CAL such as where the disorder is caused by pollutants.

Subjects with CAL may display one or more, and sometimes all, of cough, increased sputum production, dyspnea, and/or a history of exposure to risk factors for the disease. In the case of cough, increased sputum and dyspnea these may have been present for extended periods of time such as at least a month, preferably six months, more preferably at least a year and still more preferably for at least two years. Chronic cough and sputum production often precede the development of CAL and may be indicative of individuals for which the invention can be used prophylactically to prevent the development of CAL.

Subjects with CAL may have a history of exposure to pollutants, including any of those mentioned herein and at any of the levels specified herein. In particular, the subject with CAL will have a history of exposure to tobacco and/or industrial pollutants.

The subject with CAL will typically be a mature adult. For example, the subject may be from 21 to 85, preferably from 25 to 70, more preferably from 30 to 60 and even more preferably from 40 to 50 years of age. The onset of any of, or a particular, symptom mentioned herein in association with CAL, will typically have been in adulthood. For example, the subject may have been at least 20, more preferably at least 25, still more preferably at least 30 and even more preferably at least 35 years of age before they experienced a particular symptom. In particular, the symptoms associated with more advanced stages of CAL, such as any of those mentioned herein, may have their onset at such later stages of life. Subjects with a genetic predisposition to developing CAL, such as those with α₁-antitrypsin deficiency, may develop CAL earlier. For example, they may display one or more, or a particular, symptom at from 10 to 21, preferably from 12 to 18, or more preferably from 14 to 16 years of age. Alternatively, they may first show the symptom at any of the age ranges mentioned herein. The subject may have been diagnosed at any of the ages, or within any of the age ranges, specified herein. These ranges may also apply to any disorder which the subject may have and in particular those mentioned herein.

The subject may have a genetic predisposition to developing CAL and may display a family history of the condition. For example, the subject may have α₁-antitrypsin deficiency and hence be predisposed to developing CAL. Subjects at risk of developing CAL may have had low birth weights and/or a history of exposure to pollutants in the womb or in early life. The mother of the subject may be a smoker and may have continued to smoke during pregnancy. The subject may have had a history of severe childhood respiratory infection. Reduced maximal attained lung function, as measured by spirometry, may identify individuals who are at increased risk of developing CAL.

The subject may have an early stage of CAL in which the symptoms are generally moderate and may have periods of normality or of at least reduced symptoms. Alternatively, the subject may have a more developed stage of CAL in which the symptoms, and particular the reduction in FEV₁ is more pronounced. Preferably, the methods and medicaments of the invention are used, or administered at, an early stage of CAL so that they can arrest, slow or regress the increased rate in FEV₁ decline at as early a stage as possible.

CAL is typically a progressive disorder with the severity of CAL and the extent to which it has an impact on the sufferer increasing over time. Thus there may be a progressive manifestation in the symptoms of the disorder. Chronic cough is usually the first symptom to develop. It may initially be intermittent, but later may be present every day. The cough will typically be present throughout the day, rather than just at night and in the morning. In some cases, significant airflow limitation may develop without the presence of a cough. Small amounts of tenacious sputum are commonly raised after coughing bouts.

As CAL progresses the subject may experience dyspnea. The onset of dyspnea will often be one of the reasons why a human subject will first consult a physician as it can be dehabilitating and also induce anxiety. As the lung function of the subject deteriorates further, breathlessness becomes more intrusive. The subject may display wheezing and chest tightness. The dyspnea may become worse during or after exercise. Respiratory infections may also exacerbate the condition and cause increased dyspnea. Human subjects may indicate that the dyspnea progressively impairs their ability to carry out physical labour and to exert themselves.

CAL sufferers are sometimes split into categories which reflect the stage and severity of the disease. This can help define the needs of a particular subject and what course of treatment should be administered. In one classification (GOLD Executive Summary, supra) subjects are split into the following categories:
Category 0 - At risk
   Lung function, as measured by spirometry, is normal.
   Chronic symptoms (cough, sputum, production).
   Typically, human subjects will be unaware of abnormal lung function.
Category I: Mild CAL
   Mild airflow limitation.
   FEV₁/FVC < 70%.
   FEV₁ greater than, or equal to, 80% of that predicted.
   With or without chronic symptoms (cough, sputum, production).
   Human subjects may be unaware that their lung function is abnormal.
Category II: Moderate CAL
   Worsening airflow limitation.
   FEV₁/FVC < 70%.
   FEV₁ from 30 to 80% of that predicted (category can be subdivided into: IIA - FEV₁ from 50 to 80% of that predicted; and IIB - FEV₁ from 30 to 50% of that predicted).
   With, or without, chronic symptoms (cough, sputum, production, dyspnea). The symptoms may typically become more pronounced on exertion. Human subjects are likely to be aware of the condition and have sort medical advice.
Category III: Severe CAL
   Severe airflow limitation.
   FEV₁/FVC < 70%
   FEV₁ less than 30% predicted or alternatively an FEV₁ less than 50% of that predicted in conjunction with respiratory failure or clinical signs of right heart failure (respiratory failure: arterial partial pressure of oxygen (PaO₂) less than 8.0 kPa (60 mm Hg) with or without arterial partial pressure of CO₂ (PaCO₂) greater than 6.7 kPa (50 mm Hg) while breathing air at sea level).

Subject to be treated using the invention who have CAL may fall within any of the above categories. In particular, the subject may be one in categories I to III, preferably in categories II to III, and even more preferably in category III. The invention also encompasses the treatment of individuals at risk of CAL (category 0) and with an early stage of the disorder (category I).

Some of the symptoms of CAL are displayed by sufferers of other diseases. For example, a number of respiratory disorders other than CAL compromise lung function. However, these diseases may be distinguished from CAL by a full assessment of the subject and preferably by applying the relevant available guidelines for the diagnosis of CAL. The British Thoracic Society have published a set of Guidelines (Thorax 1997, 52 (Suppl. 5): S1-28) and in a preferred embodiment a subject to be treated will fall within the definition of the disorder provided by these Guidelines. In addition, the Global initiative for chronic Obstructive Lung Disease (GOLD) has published an Executive Summary (2001) outlining a strategy for the diagnosis, management and prevention of the disorder. In a preferred embodiment the subjects to be treated who have CAL will fall within the definition of the disorder provided by the Executive Summary.

CAL does not include cystic fibrosis (CF), although the invention can be used to treat both CAL and CF. Typically a subject with CAL will have a least one functional copy of the CFTR gene (unless they have been unfortunate enough to develop both CF and CAL). CAL typically has a much later onset than Cystic Fibrosis, where a sufferer is born with the defect. In CAL the decline in lung function and the onset of symptoms will occur progressively over time. In CF the decline in lung function will have a more immediate onset, earlier on in life. A subject who has CAL will often be in their thirties, forties or even fifties before they become aware that they are suffering from the disorder. The major exception to the later onset of CAL, is in those sufferers who have α₁ anti-trypsin deficiency. Such subjects will display an earlier onset of CAL, such as in their teens or twenties when CAL is diagnosed, as they will be born with a genetic condition predisposing them to CAL. Subjects who have α₁ antitrypsin deficiency and CF can readily be distinguished from each other by biochemical and genetic tests to identify the nature of the disorder.

The subject is a vertebrate animal and is a mammal. Typically the subject will be human. However, the invention also encompasses the treatment of animals with conditions which are the same as, or equivalent to, the human conditions mentioned herein. Hence, the animal will have a disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection. The animal condition may have either an underlying pathology similar to, or the same as, that of the human condition. The animal may have one or more similar symptoms or characteristics of the human condition and in particular one or more of those listed above. The animal will suffer from a condition which falls within the definition of any of these conditions listed above. The animal condition will alter mucus viscosity and/or levels in the animal

In cases where the subject is not human it may be a domestic animal or an agriculturally important animal. The animal may, for example, be a sheep, pig, cow, bull, poultry bird or other commercially farmed animal. In particular the animal may be a cow or bull and preferably is a dairy cow. The animal may be a domestic pet such as a dog, cat, bird, or rodent. In a preferred embodiment the animal may be a cat or other feline animal. The animal may be a monkey such as a non-human primate. For example, the primate may be a chimpanzee, gorilla, or orangutan. In a preferred embodiment of the invention the animal may be a horse and, for example, may be a racehorse. The animal may be a sports animal such as, for example, a greyhound.

### Subject Assessment

The present invention provides a way to improve the delivery of inhaled, nasally administered or instilled agent and in particular a therapeutic agent delivered via such a route. Thus by using a glycosaminoglycan the same amount of agent may be administered to the subject, but a higher concentration of it can be achieved at the target site. This means that less agent needs to be administered and/or a higher concentration of the agent can be achieved at the target site for a given amount of agent administered.

Subject assessment may be used to determine optimal ratios of glycosaminoglycan to agent and the increase in bioavailabilty. It may also be used to monitor the improvement of the condition of the subject. It may be used to tailor the particular therapeutic or non-therapeutic regimen to the particular needs of the subject. It may be possible to do this so that at times of particular severity of a disorder, or particular circumstances, that the treatment regimen can be adjusted appropriately. Subject assessment can be used to confirm the factor by which a particular glycosaminoglycan increases delivery of the agent.

The assessment made will be suitable for the disorder and/or the agent being administered. Ways of characterising particular disorders will be well known in the art and any suitable means for a particular disorder may be used. In the case where medicaments are to be administered to a healthy subject the particular parameter which the agent is designed to modulate may be assessed rather than the treatment of a specific condition. A number of parameters may be useful for several different disorders, such as spirometry to measure FEV₁, FVC, and/or FVC/ FEV₁ in the subject and hence assess airflow limitation. In particular spirometry may be used to assess FEV₁. Any effect of the agent on the subject and/or the disease state may be measured to confirm, and preferably quantify, the increased delivery of the drug. In particular, one of the symptoms of the disorder may be measured/assessed.

The medicaments of the invention preferably induce an improvement in the condition of the subject. The medicaments and methods may therefore be used to manage a patient suffering from, or prone to, any of the disorders mentioned herein. They may prevent, ameliorate, improve or cure the condition. They may prevent, reduce or reverse one or more of the symptoms and manifestations associated with the disorder. They will preferably also increase the feeling of well being in the subject and their quality of life. The effect of a medicament will depend on the specific therapeutic agent, but a number of examples may be provided. A medicament or method of the invention may, for example, reduce or eliminate one or more of:
- an elevated decline in forced expiratory volume (FEV₁)
- mucus hypersecretion;
- inflammation; and/or
- damage to the structure of the lung.

The ratio of FEV₁/FVC may also be improved, or any deterioration slowed or halted.

The medicaments and methods may reduce mucus plugging. They may have a mucolytic activity and/or reduce secretion and exporation of mucus. They may reduce the breakdown of the structure of the lung, such as the degradation of elastin in the airways and in the aveoulus and hence the loss of lung elasticity. They may reduce or prevent the collapse of portions of the lung and/or the development of enlarged airspaces in which air can become trapped.

In some embodiments of the invention, and in particular those where the agent is administered to a respiratory disorder such as chronic airflow limitation (CAL) the medicaments and methods may typically reduce the decline in FEV₁ by from 10 to 100%, preferably from 20 to 80%, more preferably from 30 to 60% and even more preferably from 40 to 50%. They may reduce the annual decline in FEV₁ by from 10 to 100 ml, preferably from 20 to 60 ml and even more preferably from 30 to 40ml per year. In some cases on treatment the subject will display an improvement of FEV₁ so that FEV₁ is from 25 to 100%, preferably from 40 to 100%, more preferably from 60 to 100% and even more preferably from 80 to 100% of the predicted value.

The methods and medicaments of the invention may reduce inflammation and the influx of inflammatory cells to the airways and lungs. They may also ameliorate the effects of the inflammatory cells present in the lung. Thus differential cell counts on sputum and/or lavages from the subject may display normal levels, or less elevated levels, of inflammatory cells such as lymphocytes, macrophages and/or neutrophils and in particular of neutrophils.

They may reduce migration of inflammatory cells such as neutrophils to the airways and lung. They may also reduce one or more of neutrophil functions/characteristics such as priming, activation, chemotaxis, extravasation, degranulation, respiratory burst, phagocytosis, apoptosis and/or necrosis. This may also mean that there are reduced amount of neutrophil proteins in the lung and reactive oxygen species. There may also be reduced levels of inflammatory mediators in subjects following treatment and in particular IL-8 levels. They may also cause changes in the lung such as the down regulation of adhesion molecules so that neutrophils cannot enter the lung.

The subject may also display decreased inflammation following treatment. There may be decreased levels of inflammatory mediators present in the airways of the subject. These may include proteases such as matrix metallo proteases (MMPs), cathepsins and elastase. They may also display decreased myelo-peroxidase, activated complement, MCP-1, interferons (such as, in particular, IFN-γ and interleukins such as, in particular, IL-12. Typically, IL-8 and in particular decreased IL-8 levels will be present.

In cases where the agent administered is an enzyme inhibitor, enzyme activity, compared to that in the absence of administration of an agent of the invention, may drop by 10%, preferably 20%, and more preferably 50%. It may be reduced at least two fold, preferable four fold and still more preferably at least ten fold. Conversely, where the agent is an enzyme, increased levels of enzyme, and hence enzyme activity, may be seen at the target site. For example, the increase may be two fold, three fold, five fold, ten fold or more. The increase may be from 10 to 100%, preferably from 20 to 80% and more preferably from 40 to 60%.

The lungs of the subject, or regions of them, may have decreased numbers of inflammatory cells following treatment. In particular, there may be a reduction in the number of neutrophils and/or CD8⁺ T cells moving into the airways and lung. There may also be changes in the bronchial and pulmonary vasculature, such as downregulation of adhesion molecules involved in leukocyte extravasation or decreased expression of inflammatory cytokines which can recruit inflammatory cells such as neutrophils, monocytes and T cells.

In embodiments where the therapeutic agent is a mucolytic, mucus viscosity, may be determined. Again, preferably the situation is compared prior to and post treatment. Any of the techniques discussed herein for measuring viscosity may be used such as, for example, techniques like compaction assays and assays using contortion pendulums or any other suitable rheological methods. Examples of sputum compaction assays which can be employed are described in WO94/10567 and may be employed. Examples of an assay using a torsion pendulum are described in Janmey, J. Biochem., Biophys, Methods 22:41-53, 1991, and these may also be employed to assess viscosity.

The administration of the medicaments may typically produce an improvement in the status of the subject. For example, they may result in a reduction of airway obstruction, mucus plugging, foaming and/or bubbling. The subject may show an increase in FEV₁ returning it to a value closer to that predicted for an equivalent subject not suffering from respiratory disorder. This may be accompanied by the subject experiencing increased ease in breathing, promoted expiration of mucus and reduced coughing. The subject may show increased ability to perform exercise and to physically exert themselves.

In embodiments of the invention where the medicament is an antipathogenic agent administration may decrease the incidence of respiratory infections. Thus the subject may show a decrease in infections with pathogens such as, for example, *Pseudomonas, Pneumococcus, Staphylococcus, Burkholderia, Haemophilus* and/or *Aspergillus.* In particular, the patient may show reduced incidence of infection with *Haemophilus influenza, Staphylococcus aureus, Pseudomonas aeuroginosa* and/or *Burkholderia cepacia.* Subjects where such infections were chronic may no longer be infected or have decreased load of pathogen following treatment. Pathogenic load and the nature of pathogens can be assessed using various microbiological techniques well known in the art such as staining, culturing and plating. Viral load may be decreased. Pathogenic load may be decreased by at least 10%, preferably at least 25%, more preferably by at least 50% and even more preferably by at least 75%. In some cases the agent may decrease the production of pathogenic toxins such as, for example, of endotoxin and using the medicaments of the invention a bigger decrease may be seen.

Where the agent is a gene the efficiency of delivery and expression may be assessed by well known molecular biology techniques. The proportion of cells expressing the gene may be measured and/or the proportion comprising a specific sequence. Techniques such as northern and Southern blotting may be used to detect specific sequences and their expression, as may RT-PCR and PCR. *In situ* PCR may be used. Sequences, such as actin, may be measured/detected as controls. Western blotting and antibody staining techniques may be used to detect therapeutic proteins and in particular such proteins expressed from administered nucleic acids.

In cases where the agent is a smoking cessator the subject will preferably show decreased desire for cigarettes. The subject may show increase ability for physical activity. In cases where the agent is a painkiller the subject will show a reduction in the amount of pain felt or in the duration that pain is experienced for.

The medicaments of the invention when used to administer a therapeutic agent will typically prevent or treat the disorder in question. They may ameliorate its severity and/or eliminate or reduce symptoms associated with the disorder. In particular, the therapeutic agent will have such an effect.

The medicaments may increase life expectancy such as by, for example, 1 to 2 years, preferably from 2 to 4 years and even more preferably by more than 4 years. Due to the increase in the efficiency of the delivery of the therapeutic agent the methods of the invention may mean that individuals or conditions who/which showed no improvement, or only minimal improvement, with the therapeutic agent on its own, may show improvement, or elevated improvement, when the methods of the invention are employed to increase delivery.

The effect of the therapeutic agent will depend on the nature of the particular therapeutic agent and the assessment protocol will typically be based on what the effect of the agent is known to be.

In some embodiments, labeled molecules may be administered to help assess the efficacy of the methods of the invention. For example, fluorescently or radioactively labeled molecules may be employed. In some cases, the labeled molecule may not be a therapeutic agent. In others, a labeled version of the therapeutic agent may be employed. Such methods may be used to assess delivery in both *in vitro* model systems and *in vivo* and may be employed to decide on the amount and/or ratio of therapeutic agent and glycosaminoglycan. They may also be used in imaging.

### Compositions

Also referred to is a composition comprising a glycosaminoglycan, or a salt thereof and an agent. In particular, the agent will be a therapeutic agent. In such cases the composition will typically be a pharmaceutical composition. These may typically be in a solid or liquid form. It may be in the form of a powder. It may be lyophilised or frozen. It may also comprise other components such as water or a buffer. The composition may take the form of an aqueous solution comprising both the agent and the glycosaminoglycan or salt. The composition may comprise an aqueous suspension with either, or both, the glycosaminoglycan or salt and the agent being present in, or as, the particles of the suspension. In compositions where both a glycosaminoglycan or salt and an agent are present, they may be present as separate particles or in the same particles.

The medicaments of the invention may take a variety of forms. They may be in the form of powders, powder microspheres, solutions, suspensions, gels, nano-particle suspensions, liposomes, emulsions or microemulsions. The compositions may comprise a glycosaminoglycan or salt and/or an agent dissolved or suspended in liquids other than water, for example, they may be dissolved or suspended in solvents such as CFC or HFA.

The composition may also comprise stabilising agents or preservatives. It may comprise agents which allow the product to be frozen without losing enzyme activity such as glycerol. The composition may be formed, for example, by mixing powders of glycosaminoglycan and the therapeutic agent. The therapeutic agent and the glycosaminoglycan or salt may be any of those mentioned herein.

### Administration and Pharmaceutical compositions

The glycosaminoglycan or physiologically acceptable salt may be administered simultaneously, separately or sequentially to the therapeutic agent. Thus the two may be administered in the same medicament or as two separate medicaments and may be given at the same time, one after the other or separately. In embodiments where the two are to be administered separately, preferably the glycosaminoglycan, or salt will be administered prior to, or at the same time, as the therapeutic agent.

The glycosaminoglycan and therapeutic agent may typically be administered from one week to one minute apart, preferably from two days to one hour apart, even more preferably from 24 hours to 2 hours apart and still more preferably from 12 to 4 hours apart. The two may be administered from one minute to one hour apart, preferably from 10 minutes to 30 minutes apart and even more preferably from 15 to 25 minutes apart. The two may be administered, for example, from one minute to fifteen minutes apart or at a shorter interval such as from 10 seconds to one minute and preferably from 30 seconds to one minute apart. The two may be administered in sequence, one immediately after the other.

The two will be administered by inhalation, instillation and/or intranasally preferably via the same route. In an especially preferred embodiment of the invention both will be administered via inhalation. The two may be administered together or separately. Preferably the two will be administered by the same route either as separate medicaments or in the same medicament, typically they will be in separate medicaments. The glycosaminoglycan or salt and the therapeutic agent may be provided in two separate compositions, but may, for example, be mixed prior to addition to a delivery device or in the delivery device. The delivery device may have means to regulate the amount of each delivered to the subject. It may have means to mix the two or deliver each separately.

In some embodiments of the invention one or both of the glycosaminoglycan and agent may be administered via instillation. Typically, instillation will involve the insertion of an artificial airway through which a composition of the invention is instilled. In such cases, typically the composition will be a liquid composition. Administration will typically involve drawing up the composition into a syringe or similar device and then expelling the medicament through the artificial airway into the pulmonary tract of the subject. Usually, instillation will be used in an emergency situation and/or where the subject is unconscious. Typically volumes such as from 1 to 20 ml, preferably from 2 to 10 ml, and even more preferably from 3 to 6 ml will be instilled. Any method for delivery into the pulmonary tract may be used to deliver the medicaments of the invention.

The medicaments and compositions of the present invention may be prepared by formulating the active agents, i.e. the glycosaminoglycan or salt and/or the therapeutic agent, with a standard pharmaceutically acceptable carrier and/or excipient as is routine in the pharmaceutical art. The exact nature of the formulation will depend upon several factors including the particular glycosaminoglycan, salt or therapeutic agent employed and the desired route of administration. Suitable types of formulation are fully described in Remington's Pharmaceutical Sciences, 19^{th} Edition, Mack Publishing Company, Eastern Pennsylvania, USA.

The necessary dose to be administered will normally be determined by a physician. The dose of glycosaminoglycan administered may, for example, 0.01mg to 5g, preferably from 0.1 mg to 2.5g, more preferably from 1 mg to 1g, even more preferably from 10 mg to 500mg , still more preferably from 50 mg to 250 mg and even more preferably from 100 mg to 200 mg. These doses will typically be given once, twice or three times a day and will preferably be given once or twice a day and more preferably will be given twice a day. For a heparin or salt the dose will typically be in the range of from 10 to 10,000 units per/kg body weight, preferably 100 to 10,000 units per/kg body weight, more preferably from 200 to 5000 units/kg body weight, even more preferably from 500 to 2000 units per/kg, and still more preferably from 1,000 to 1,500 units per/kg.

Typically the dose of the therapeutic agent will either be the normal amount of the therapeutic agent which is a typically administered in the absence of glycosaminoglycan if it is desired to have an overall higher amount of agent delivered to the target or will be less than the normal amount. Thus it may typically be one half, quarter, fifth, tenth or less of the amount which would have been administered in the absence of glycosaminoglycan. It may, for example, be less than 60%, preferably less than 30%, more preferably less than 15% and even more preferably less than 5% than the amount which would have been given in the absence of glycosaminoglycan. The amount of agent may, for example, be the same as glycosaminoglycan. The number of times a day, week or month that the therapeutic agent is administered in comparison to without glycosaminoglycan may be also be reduced such as by a half, a quarter or more.

A therapeutically effective amount of the therapeutic agent will be administered. Thus taking into account the presence of the glycosaminoglycan the amount of therapeutic agent will be therapeutically effective. The dose of the therapeutic agent may be determined according to various parameters, especially according to the substance used; the age, weight and condition of the patient to be treated; the route of administration; and the required regimen. A physician will be able to determine the required route of administration and dosage for any particular patient. A typical daily dose is from about 0.1 to 50 mg per kg of body weight, according to the activity of the specific therapeutic agent, the age, weight and conditions of the subject to be treated, the type of disorder. Preferably, daily dosage levels are from 5 mg to 2 g. However, dosages will depend on the particular therapeutic agent.

The length of treatment may typically be from a day, one week, two weeks, a month, six months a year or more. In many cases the subject will remain on the medicaments of the invention permanently or for extended periods. This may in particular be the case where the subject has a genetic defect, such as where the subject has cystic fibrosis or α₁-antitrypsin inhibitor deficiency, and hence permanently has the condition. This may also be the case where one of the causative factors in the disorder to be treated is something that the subject is continuously exposed to and cannot avoid, or does not wish to minimise exposure to. The subject may be a tobacco smoker, but not give up smoking tobacco. In cases where the therapeutic agent is insulin or a contraceptive it may also may be administered for extended periods.

In cases where the respiratory disorder is more short lived, or occurs periodically, the length of administration may be shorter and may be linked to the duration of the disorder. Thus, for example, where the condition is a cold or the flu, once it has been resolved administration of the medicament may be ceased or reduced. The administration schedule may be coordinated so that higher levels of the medicaments are administered, or initiation of administration begins, at times when the disorder has increased severity. Thus in respiratory disorders such as, for example, cystic fibrosis or pneumonia this may be when the subject has an increase in airway obstruction, infection and/or inflammation. The medicament may also be given prior to exercise or other physical exertion.

Preferably the medicaments of the invention may be administered via inhalation and hence will be given by the nose or mouth. Inhaled delivery includes intra-nasal delivery. They also may be administered via instillation, such as through an artificial airway. Suitable methods for formulating and preparing medicaments to be administered via inhalation, instillation and intranasally are well known in the art and may be employed in the present invention. For inhalation, intranasal or instillation therapy the medicament may be in a solution useful for administration by nebuliser or other liquid aerosol device, metered dose inhalers, or in a form suitable for a dry powder inhaler. The medicament may be present in a blister pack or breakable capsule. The devices suitable for administering the medicaments of the invention include in some cases, for example where the subject is a child, a spacer which may be used in conjunction with the inhaler to help ensure effective delivery.

In some preferred embodiments, the medicaments of the present invention may be formulated as aerosols. The formulation of pharmaceutical aerosols is routine to those skilled in the art, see for example, Sciarra, J. in Remington's Pharmaceutical Sciences (*supra*). The agents may be formulated as solution aerosols, dispersion or suspension aerosols of dry powders, emulsions or semisolid preparations. The aerosol may be delivered using any propellant system known to those skilled in the art. The aerosols may be applied to the upper respiratory tract, for example by nasal inhalation, or to the lower respiratory tract or to both. The glycosaminoglycan, salt or therapeutic agent may delivered using liposomes and nano-particle delivery methods. Liposomes, particularly cationic liposomes, may be used in carrier formulations. Where the therapeutic agent is intended for gene therapy purposes it may, for example, take the form of a virus, liposome or other suitable vector.

In cases where the glycosaminoglycan and/or therapeutic agent are administered in the form of particles or droplets, the particle/droplet size and/or other properties of the particle/droplet may be chosen to ensure that the particles are delivered to a particular region of the respiratory tract. For example, they may be designed to reach only the upper or lower parts of the respiratory tract. In cases where the glycosaminoglycan, salt or therapeutic agent are delivered in an aqueous form preferably the solution will be isotonic to help ensure effective delivery to the subject.

In many embodiments, the particle size of the medicament may be chosen on the basis of the desired part of the respiratory tract which it is desired to administer the medicament to. In particular particles with a diameter of 10µM are thought to be effective in reaching the lower parts of the respiratory tract and hence may be employed where such a site is the desired target for the medicaments. In embodiments, where it is desired to deliver the medicament to the lower parts of the respiratory tract, such as alveoli for example, the diameter of the particles administered may be less than 10µM, preferably less than 8µM, more preferably less than 6µM and even more preferably less than 4µM. In a preferred embodiment the particles may have a diameter of 3µM or less and more preferably may have a diameter of 2µM or less. In an especially preferred embodiment the particles will have a diameter of from 3 to 5µM. In some cases the particles administered may be less than 1000 nm, preferably less than 500 nm, more preferably less than 250 nm and still more preferably less than 100 nm in diameter. The sizes may refer to particles of solid matter or droplets of solutions and suspensions.

The size of particles necessary to penetrate to a specific part of the respiratory tract will be known in the art and hence the particle size can be chosen to suit the target size. Techniques such as milling may be used to produce the very small particles necessary. In some cases the desired part of the respiratory tract may be the upper respiratory tract and hence larger particles sizes may be employed. The density of the particles and their shape may also be chosen to facilitate their delivery to the desired site.

In cases where the medicament is to be administered via the nose it may be, for example, in the form of nasal spray. The spray may be administered, for example, using an atomizer or nebulizer. In some cases the medicament may be in the form of nasal drop. These may be administered using a via a medicine dropper. For further discussion on nasal dosage forms see Remington's Pharmaceutical Sciences (*Supra*). Medicaments administered via the nose may include pH adjusters, emulsifiers or dispersing agents, preservatives, surfactants, gelling agents, or buffering agents. Most preferably the nasal dosage form will be isotonic with nasal secretions.

Medicaments for use in accordance with the present invention, may include, in addition to the active ingredients, a pharmaceutically acceptable excipient, carrier, buffer, stabiliser or other materials well known to those skilled in the art. In particular they may include a pharmaceutically acceptable excipient. Such materials should be non-toxic and should not interfere with the efficacy of the active ingredient. The precise nature of the carrier or other material will depend on the route of administration. Suitable pharmaceutical carriers are described in Remington's Pharmaceutical Sciences (*supra*)*.*

The medicaments may include various constituents to optimise their suitability for the particular delivery route chosen. The viscosity of the medicaments may be maintained at a desired level using a pharmaceutically acceptable thickening agent. Thickening agents that can be used include methyl cellulose, xanthan gum, carboxymethyl cellulose, hydroxypropyl cellulose, carbomer, polyvinyl alcohol, alginates, acacia, chitosans and combinations thereof. The concentration of the thickening agent will depend upon the agent selected and the viscosity desired.

In some embodiments, and in particular where intranasal delivery is to be used, the medicaments may comprise a humectant. This may help reduce or prevent drying of the mucus membrane and to prevent irritation of the membranes. Suitable humectants include sorbitol, mineral oil, vegetable oil and glycerol; soothing agents; membrane conditioners; sweeteners; and combinations thereof.

The medicaments may comprise a surfactant. Suitable surfactants include non-ionic, anionic and cationic surfactants. Examples of surfactants that may be used include, for example, polyoxyethylene derivatives of fatty acid partial esters of sorbitol anhydrides, such as for example, Tween 80, Polyoxyl 40 Stearate, Polyoxy ethylene 50 Stearate, fusieates, bile salts and Octoxynol.

The medicaments of the present invention may be delivered by any device adapted to introduce one or more therapeutic composition(s) into the upper and/or lower respiratory tract. In some preferred embodiments, the devices of the present invention may be metered-dose inhalers. The devices may be adapted to deliver the therapeutic compositions of the invention in the form of a finely dispersed mist of liquid, foam or powder. The device may use a piezoelectric effect or ultrasonic vibration to dislodge powder attached on a surface such as a tape in order to generate mist suitable for inhalation. The devices may use any propellant system known to those in the art including, but not limited to, pumps, liquefied-gas, compressed gas and the like.

Devices of the present invention typically comprise a container with one or more valves through which the flow of the therapeutic composition travels and an actuator for controlling the flow. Suitable devices for use in the present invention may be seen in, for example, in Remington's Pharmaceutical Sciences (*supra*)*.* The devices suitable for administering the medicaments of the invention include inhalers and nebulisers or other liquid aerosol devices such as those typically used to deliver steroids to asthmatics.

Various designs of inhalers are available commercially and may be employed to deliver the medicaments of the invention. These include the Accuhaler, Aerohaler, Aerolizer, Airmax, Autohaler, Clickhaler, Diskhaler, Easi-breathe inhaler, Fisonair, Integra, Jet inhaler, Miat-haler, Novolizer inhaler, Pulvinal inhaler, Rotahaler, Spacehaler, Spinhaler, Syncroner inhaler and Turbohaler devices. A number of formulation techniques which produce particularly desirable particles are known in the art and may be employed. For example the nanocrystal, pulmosol and pulmosphere technologies may be employed.

Also referred to is a pharmaceutical composition comprising:
- a glycosaminoglycan, or a physiologically acceptable salt thereof;
- a therapeutic agent; and
- a pharmaceutically acceptable excipient, carrier or diluent.

The excipient may be any of those mention herein, or any of the carriers. The composition may take the form of any of the medicaments discussed herein or any of the characteristics or features specified herein for them. The therapeutic agent and glycosaminoglycan or salt may be any of those discussed herein.

### Kits

Also referred to is a kit comprising:
- a glycosaminoglycan, or a physiologically acceptable salt thereof;
- instructions for the administration of the glycosaminoglycan or salt as described herein to a subject by inhalation, intranasally and/or via instillation, where the subject is being treated with a therapeutic agent which is also being administered by inhalation, intranasally and/or via instillation; and
- packaging.

Also referred to is a kit comprising:
- a therapeutic agent;
- instructions for the administration of the therapeutic agent to a subject by inhalation, intranasally and/or via instillation where the subject is also being treated with a glycosaminoglycan, or a physiologically acceptable salt thereof as described herein which is being administered by inhalation, intranasally and/or via instillation; and
- packaging.

Also referred to is a kit comprising:
- a glycosaminoglycan, or a physiologically acceptable salt thereof;
- instructions for the administration of the glycosaminoglycan or salt as described herein to a subject by inhalation, intranasally and/or via instillation where the subject is being treated with a therapeutic agent which is being administered by inhalation, intranasally and/or via instillation and the two are administered sequentially, simultaneously or separately; and
- packaging.

Also referred to is a kit comprising:
- a therapeutic agent;
- instructions for the administration of the therapeutic agent to a subject by inhalation, intranasally and/or via instillation where the subject is being treated with a glycosaminoglycan, or a physiologically acceptable salt thereof as described herein which is being administered by inhalation, instranasally and/or via instillation and the two are administered sequentially, simultaneously or separately; and
- packaging.

The subject will be suffering from a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection.

The instructions of the kits may take the form of a label on the packing. The kit may additionally comprise means for administering the glycosaminoglycan or salt and/or the agent such as any of those discussed herein. The agent, subject, method of administration and any of the other parameters of the kit may be as described elsewhere herein.

The following Examples illustrate the invention.

### Example 1: Barrier Assay on Victor Fluorometer

CAL sputum samples were thawed at room temperature and homogenised using a syringe (no needle). Samples were split and portions (0.12-0.20 g) weighed in small roundbottomed tubes. Tubes were centrifuged in a microfuge to sediment sputum. Mucolytic was added to sputum on a 10% volume to weight basis (e.g. 13 µl/0.13 g sputum). The mucolytic was therefore diluted by a factor of 10 upon addition to sputum. The mucolytics assessed were DNase (at a concentration of 2.9 µg/ml of a stock of activity 2500 kunits per mg) and unfractionated heparin (at a concentration of 10 mg/ml of a stock of 160 USP/mg - a sodium heparin salt from porcine intestinal mucosa from Calbiochem, Catalogue number 375095, was used). A control portion of sputum was treated with PBS on a 10% volume to weight basis. A further control with the non-glycosaminoglycan dextran sulphate was also performed (at a concentration of 10 mg/ml - a sodium dextran salt from sigma, Catalogue number D-7307, was used). The mucolytic was thoroughly mixed with the sputum by vortexing and passing up and down a 19-gauge needle.

The lower wells of a Transwell chamber contained 25µl of phosphate buffered saline and this was separated from the upper wells by an 8µm pore-size polycarbonate filter (placed shiny side down). The upper section of the chamber was placed on top of the filter and screwed down tightly. Carboxylate-modified fluorescent beads (F-8811, Molecular Probes) were sonicated for 5 mins to disperse them and beads added to sputum samples in a 1:1 ratio, e.g. 150µl beads added to 0.15g sputum sample. The mixture was vortexed to mix and 20µl of each sample added to the upper wells of the chamber. Each sample was added to the chamber in quadruplicate. The chamber was centrifuged for 5 mins at 1000 rpm to remove air bubbles, then placed inside a black plastic bag and incubated in a humid chamber at 37°C, 900rpm, for 4 hrs.

Following incubation, the chamber was centrifuged for 5 mins at 1000rpm to force migrated beads to the bottom of the lower wells. The upper chamber and gasket were removed in one piece to minimise leakage. The contents of the lower wells (25µl) were transferred to a Fluoro-NUNC plate and 175µl PBS added per well. The plate was read using Victor fluorometer with the 'Fluospheres' protocol (excitation 485nm, emission 535nm).

The results obtained are shown in Figure 1. The mucolytic effects of heprain were greater and less variable than those of DNase. A Bonferri multiple comparisons test showed that the effect of 10 mg/ml heparin was significantly greater than that of the PBS control (P<0.01). However, the effects of DNase and 10 mg/ml dextran sulphate (negative control) were not significantly different when compared to the PBS control (P>0.05).

The effect of chondroitin sulphate on microsphere transport in CF sputum was also assessed using the same conditions as those described above. A 70:30 mixture of chondroitins A and C was tested at concentralcons of 0, 0.1, 1 and 10 mg/ml (a sodium salt of chondroilin sulphate A and C from bovine trachea obtained from sigma, Catalogue number C-3788 was used). A control containing sputum treated with PBS alone was again run. The results obtained are shown in the bottom panel of Figure 1. Chondroitin sulphate was found to significantly increase transport of the microspheres.

### Example 2: Atomic Force Microscopy (AFM) imaging of DNA

Calf thymus DNA (0.1 mg/ml) was treated with heparin at various concentrations (0.1, 1, 10 and 100 µg/ml). Solutions were prepared in 0.2 :m filtered, DNase and RNase free water. 10 :1 of sample was added to freshly cleaved ruby muscovite mica and dried prior to AFM imaging. AFM was performed in air under ambient conditions using a TopoMetrix TMX2000 Scanning Probe Microscope (ThermoMicroscopes, Bicester, UK) with a 70'70'12 mm tripod piezoelectric scanner. Topography measurements, in contact mode, were made using a "V"-shaped silicon nitride cantilever (length 200 nm, nominal spring constant (K) 0.21 Nm-1; Part. No. 1530-00, ThermoMicroscopes, Santa Clara, California, USA) bearing an integrated standard profile tip.

The results are shown in Figures 2 and Figure 3. In Figure 2, Panel A = 0.1 mg/ml DNA and Panel B = 0.1 mg/ml DNA + 0.1 mg/ml heparin. The addition of heparin to DNA was shown to alter the structure of the DNA network, increasing the average pore size from 180 (SD 30) :m to 780 (SD 150) :m

Figure 3 shows results with lower concentrations of heparin. Panel A shows the results for untreated DNA: Panel B for DNA treated with 0.1 µg/ml heparin; Panel C for DNA treated with 1 µg/ml heparin; and Panel D for DNA treated with 10 µg/ml heparin.

## Claims

1. A glycosaminoglycan selected from chondroitin sulphates A to E, heparin, heparin sulphate, heparan, heparan sulphate and keratan sulphate or a physiologically acceptable salt of such a glycosaminoglycan for use in a method of treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is being treated with a therapeutic agent;
- the glycosaminoglycan or salt and the therapeutic agent are administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

2. A therapeutic agent for use in a method of treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is also administered a glycosaminoglycan selected from chondroitin sulphates A to E, heparin, heparin sulphate, heparan, heparan sulphate and keratan sulphate or a physiologically acceptable salt of such a glycosaminoglycan;
- the glycosaminoglycan or salt and the therapeutic agent are administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

3. A glycosaminoglycan for use according to claim 1 or a therapeutic agent for use according to claim 2, wherein the subject has a respiratory tract infection.

4. A glycosaminoglycan or therapeutic agent for use according to claim 3, wherein the infection is a bacterial or viral infection.

5. A glycosaminoglycan or therapeutic agent for use according to claim 4 wherein the viral infection is influenza or a cold.

6. A glycosaminoglycan for use according to any one of claims 1 and 3 to 5 or a therapeutic agent for use according to any one of claims 2 to 5, wherein the subject has chronic airflow limitation (CAL).

7. A glycosaminoglycan for use according to any one of claims 1 and 3 to 6 or a therapeutic agent for use according to any one of claims 2 to 6, wherein the subject is human.

8. A glycosaminoglycan for use according to any one of claims 1 and 3 to 7 or a therapeutic agent for use according to any one of claims 2 to 7, wherein the glycosaminoglycan or the physiologically acceptable salt comprises repeating disaccharide units of general formula (1)
-[A-B]- (1)
wherein:
- each A is the same or different and represents a moiety of formula (i) or (ii) 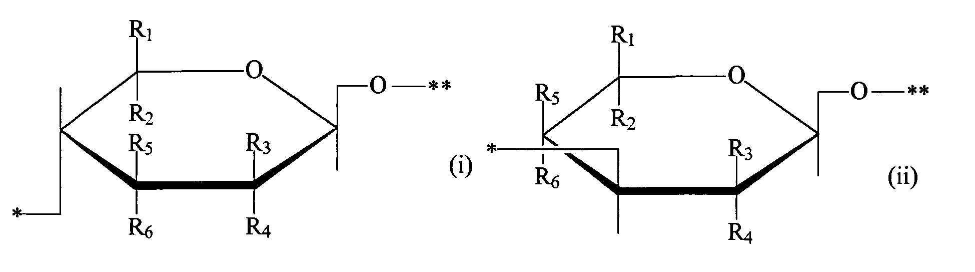
wherein:
- one of R₁ and R₂ is hydrogen, and the other is -CO₂H, -SO₃H or -CH₂OR wherein R is hydrogen or -SO₃H;
- one of R₃ and R₄ is hydrogen, and the other is -OR wherein R is hydrogen or SO₃H;
- one of R₅ and R₆ is hydrogen, and the other is -OH;
- * represents a direct bond to an adjacent hydrogen atom or B moiety; and
- ** represents a direct bond to an adjacent B moiety;
each B is the same or different and represents a moiety of formula (iii) or (iv) 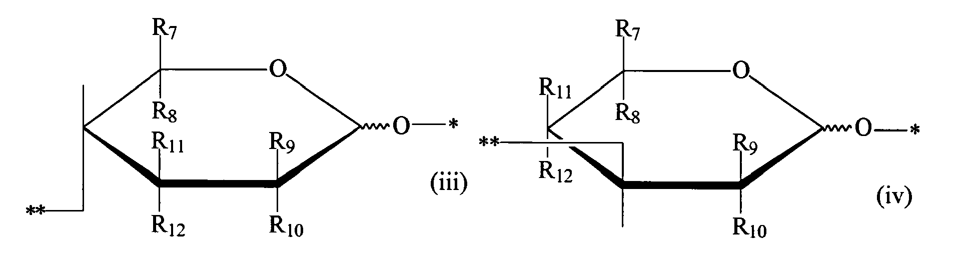 wherein:
- one of R₇ and R₈ is hydrogen and the other is -CH₂OH or -CH₂OSO₃H;
- one of R₉ and R₁₀ is hydrogen and the other is -NHAc, -NH₂ or -NHSO₃H;
- one of R₁₁ and R₁₂ is hydrogen and the other is -OH or -OSO₃H;
- 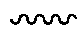 indicates a bond in either stereochemical orientation;
- * represents a direct bond to a hydrogen atom or an adjacent A moiety; and
- ** represents a direct bond to an adjacent A moiety.

9. A glycosaminoglycan for use according to any one of claims 1 and 3 to 8 or a therapeutic agent for use according to any one of claims 2 to 8 wherein the glycosaminoglycan is:
(a) a heparin or a derivative thereof; or
(b) chondroitin sulphate A, chondroitin sulphate C, chondroitin sulphate D, chondroitin sulphate E, keratan sulphate, heparan sulphate or a derivative of any thereof.

10. A glycosaminoglycan for use according to any one of claims 1 and 3 to 9 or a therapeutic agent for use according to any one of claims 2 to 9, wherein the sodium salt of heparin or heparin sulphate is used.

11. A glycosaminoglycan for use according to any one of claims 1 and 3 to 10 or a therapeutic agent for use according to any one of claims 2 to 10, wherein the glycosaminoglycan or salt:
(a) retains anti-coagulant activity; and/or
(b) has not been subjected to fragmentation and/or depolymerisation.

12. A glycosaminoglycan for use according to any one of claims 1 and 3 to 11 or a therapeutic agent for use according to any one of claims 2 to 11, wherein the ratio of the amount of glycosaminoglycan or salt to the amount of therapeutic drug administered is from 500:1 to 1:500.

13. A glycosaminoglycan or therapeutic agent for use according to claim 12, wherein the ratio is from 25:1 to 1:25.

14. A glycosaminoglycan for use according to any one of claims 1 and 3 to 13 or a therapeutic agent for use according to any one of claims 2 to 13, wherein
(a) the glycosaminoglycan or salt and the therapeutic agent are administered at the same time;
(b) the glycosaminoglycan or salt is administered before or after the therapeutic agent; and/or
(c) the glycosaminoglycan or salt and the therapeutic agent are administered as separate medicaments.

15. A glycosaminoglycan for use according to any one of claims 1 and 3 to 14 or a therapeutic agent for use according to any one of claims 2 to 14, wherein the therapeutic agent is selected from a bronchodilator, a mucolytic, a mucokinetic, an anti-inflammatory, a protease inhibitor, α1-antitrypsin, a mucoregulator, an anti-pathogenic agent, a gene therapy vector and an antioxidant and a steroid.

16. A glycosaminoglycan or therapeutic agent for use according to claim 15, wherein the anti-pathogenic agent is an antibiotic, an antiviral agent or an antifungal agent.

17. A glycosaminoglycan or therapeutic agent for use according to claim 15, wherein the protease inhibitor is selected from an inhibitor of elastase, a cathepsin or a matrix metallo proteinase (MMP).

18. A glycosaminoglycan for use according to any one of claims 1 and 3 to 17 or a therapeutic agent for use according to any one of claims 2 to 17, wherein the subject has an FEV1 of less than 75% of the expected value for an equivalent subject not having the disorder.

19. A glycosaminoglycan for use according to any one of claims 1 and 3 to 18 or a therapeutic agent for use according to any one of claims 2 to 18, wherein the respiratory disease or disorder is **characterised by** mucus hypersecretion or elevated mucus viscosity.

20. A glycosaminoglycan for use according to any one of claims 1 and 3 to 19 or a therapeutic agent for use according to any one of claims 2 to 19 wherein the medicament is a dry powder.

21. Products comprising a glycosaminoglycan selected from chondroitin sulphates A to E, heparin, heparin sulphate, heparan, heparan sulphate and keratan sulphate, or a physiologically salt of such a glycosaminoglycan and a therapeutic agent for simultaneous, separate or sequential use in the treatment of a human subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the glycosaminoglycan or salt and the therapeutic agent are to be administered via inhalation, intranasally and/or via instillation;
- the glycosaminoglycan or salt are as defined in any one of claims 1 and 9 to 12 and have an average molecular weight of from 12 to 18 kd;
- the agent is to be delivered to the respiratory tract;
- the mucus or the other pulmonary secretions contains extracellular genomic DNA which is compromising lung function; and
- the glycosaminoglycan increases delivery of the therapeutic agent.

22. Use of a glycosaminoglycan selected from chondroitin sulphates A to E, heparin, heparin sulphate, heparan, heparan sulphate and keratan sulphate or a physiologically acceptable salt of such a glycosaminoglycan for the manufacture of a medicament for treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is being treated with a therapeutic agent;
- the glycosaminoglycan or salt and the therapeutic agent are to be administered via inhalation, intranasally and/or via instillation;
- the agent is to be delivered to the respiratory tract;
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

23. Use of a therapeutic agent for the manufacture of a medicament for treating a mammalian subject with a respiratory disorder or disease which comprises increased levels and/or viscosity of mucus or other pulmonary secretions, where the disease or disorder is selected from chronic airflow limitation (CAL), pneumonia, sinusitis, sinus congestion, cystic fibrosis, asthma, bronchitis, tracheobronchitis, bronchiectasis, tuberculosis, fungal infection, viral infection or a respiratory tract infection, wherein:
- the subject is also administered a glycosaminoglycan selected from chondroitin sulphates A to E, heparin, heparin sulphate, heparan, heparan sulphate and keratan sulphate or a physiologically acceptable salt of such a glycosaminoglycan;
- the glycosaminoglycan or salt and the therapeutic agent are to be administered via inhalation, intranasally and/or via instillation;
- the agent is to be delivered to the respiratory tract
- the said glycosaminoglycan or salt thereof has an average molecular weight of from 12 to 18 kd;
- the mucus or the other pulmonary secretions contains extracellular genomic DNA which is compromising lung function; and
- the glycosaminoglycan increases delivery of the therapeutic agent.

24. Products for use according to claim 21 or a use according to claim 22 or 23 wherein:
- the therapeutic agent is as defined in any one of claims 12 to 14; and/or
- the ratio of glycosaminoglycan or salt to therapeutic agent is as defined in claim 12 or 13.

25. Products for use according to claim 21 or 24 or use according to any one of claims 22 to 24, wherein the subject is as defined in any one of claims 3 to 7.

## Patentansprüche

1. Glycosaminoglycan, ausgewählt aus Chondroitinsulfaten A bis E, Heparin, Heparinsulfat, Heparan, Heparansulfat und Keratansulfat, oder ein physiologisch annehmbares Salz eines solchen Glycosaminoglycans zur Verwendung in einem Verfahren zur Behandlung eines Säugetiersubjektes mit einer Atemwegsstörung oder -erkrankung, die erhöhte Level und/oder Viskosität von Mukus oder anderen pulmonalen Sekretionen umfasst, wobei die Erkrankung oder Störung aus chronischer Einschränkung des Luftflusses (chronic airflow limitation (CAL)), Pneumonie, Sinusitis, Sinuskongestion, zystischer Fibrose, Asthma, Bronchitis, Tracheobronchitis, Bronchiektasie, Tuberkulose, Pilzinfektion, Virusinfektion oder einer Respirationstraktinfektion ausgewählt ist, wobei:
- das Subjekt mit einem Therapeutikum behandelt wird;
- das Glycosaminoglycan oder Salz und das Therapeutikum durch Inhalation, intranasal und/oder durch Instillation verabreicht werden;
- das Mittel an den Respirationstrakt abzugeben ist;
- das Glycosaminoglycan oder Salz davon ein durchschnittliches Molekulargewicht von 12 bis 18 kd hat;
- der Mukus oder die anderen pulmonalen Sekretionen extrazelluläre genomische DNA enthalten, welche die Lungenfunktion gefährdet, und
- das Glycosaminoglycan eine Abgabe des Therapeutikums erhöht.

2. Therapeutikum zur Verwendung in einem Verfahren zur Behandlung eines Säugetiersubjektes mit einer Atemwegsstörung oder -erkrankung, die erhöhte Level und/oder Viskosität von Mukus oder anderen pulmonalen Sekretionen umfasst, wobei die Erkrankung oder Störung aus chronischer Einschränkung des Luftflusses (chronic airflow limitation (CAL)), Pneumonie, Sinusitis, Sinuskongestion, zystischer Fibrose, Asthma, Bronchitis, Tracheobronchitis, Bronchiektasie, Tuberkulose, Pilzinfektion, Virusinfektion oder einer Respirationstraktinfektion ausgewählt ist, wobei:
- dem Subjekt auch ein Glycosaminoglycan, ausgewählt aus Chondroitinsulfaten A bis E, Heparin, Heparinsulfat, Heparan, Heparansulfat und Keratansulfat, oder ein physiologisch annehmbares Salz eines solchen Glycosaminoglycans verabreicht wird;
- das Glycosaminoglycan oder das Salz und das Therapeutikum durch Inhalation, intranasal und/oder durch Instillation verabreicht werden;
- das Mittel an den Respirationstrakt abzugeben ist;
- das Glycosaminoglycan oder Salz davon ein durchschnittliches Molekulargewicht von 12 bis 18 kd hat;
- der Mukus oder die anderen pulmonalen Sekretionen extrazelluläre genomische DNA enthalten, welche die Lungenfunktion gefährdet, und
- das Glycosaminoglycan eine Abgabe des Therapeutikums erhöht.

3. Glycosaminoglycan zur Verwendung gemäß Anspruch 1 oder ein Therapeutikum zur Verwendung gemäß Anspruch 2, wobei das Subjekt eine Respirationstraktinfektion hat.

4. Glycosaminoglycan oder Therapeutikum zur Verwendung gemäß Anspruch 3, wobei die Infektion eine Bakterien- oder Virusinfektion ist.

5. Glycosaminoglycan oder Therapeutikum zur Verwendung gemäß Anspruch 4, wobei die Virusinfektion Influenza oder eine Erkältung ist.

6. Glycosaminoglycan zur Verwendung gemäß einem der Ansprüche 1 und 3 bis 5 oder Therapeutikum zur Verwendung gemäß einem der Ansprüche 2 bis 5, wobei das Subjekt eine chronische Einschränkung des Luftflusses (chronic airflow limitation (CAL)) hat.

7. Glycosaminoglycan zur Verwendung gemäß einem der Ansprüche 1 und 3 bis 6 oder Therapeutikum zur Verwendung gemäß einem der Ansprüche 2 bis 6, wobei das Subjekt ein Mensch ist.

8. Glycosaminoglycan zur Verwendung gemäß einem der Ansprüche 1 und 3 bis 7 oder Therapeutikum zur Verwendung gemäß einem der Ansprüche 2 bis 7, wobei das Glycosaminoglycan oder das physiologisch annehmbare Salz Disaccharid-Wiederholungseinheiten der allgemeinen Formel (1) umfasst
-[A-B]- (1)
worin:
- jedes A gleich oder unterschiedlich ist und eine Gruppierung der Formel (i) oder (ii) darstellt 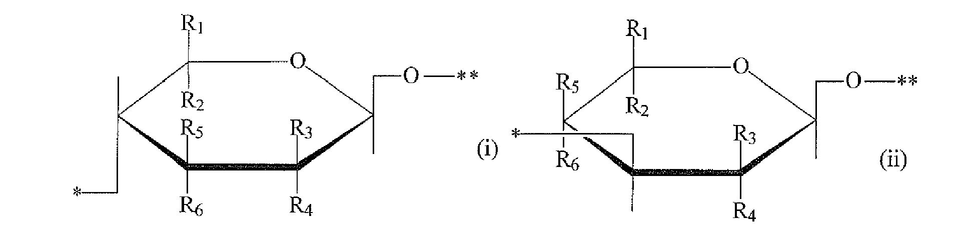
worin:
- eins von R₁ und R₂ Wasserstoff ist und das andere -CO₂H, -SO₃H oder -CH₂OR, worin R Wasserstoff oder -SO₃H ist, ist;
- eins von R₃ und R₄ Wasserstoff ist und das andere -OR, worin R Wasserstoff oder SO₃H ist, ist;
- eins von R₅ und R₆ Wasserstoff ist und das andere -OH ist;
- * eine direkte Bindung zu einem benachbarten Wasserstoffatom oder einer B-Gruppierung darstellt und
- ** eine direkte Bindung zu einer benachbarten B-Gruppierung darstellt;
jedes B gleich oder unterschiedlich ist und eine Gruppierung der Formel (iii) oder (iv) darstellt 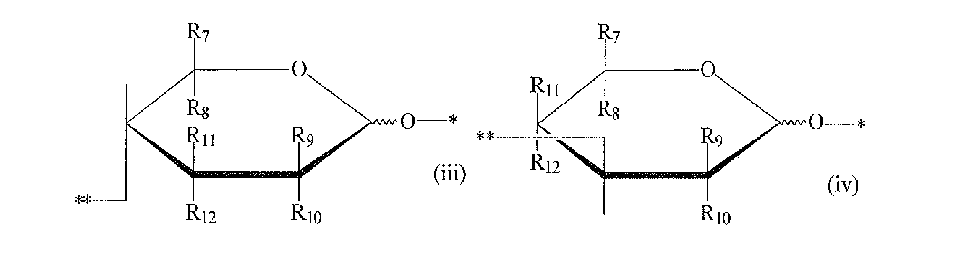 worin:
- eins von R₇ und R₈ Wasserstoff ist und das andere -CH₂OH oder -CH₂OSO₃H ist;
- eins von R₉ und R₁₀ Wasserstoff ist und das andere -NHAc, -NH₂ oder -NHSO₃H ist;
- eins von R₁₁ und R₁₂ Wasserstoff ist und das andere -OH oder -OSO₃H ist;
- 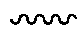 eine Bindung in jeder stereochemischen Orientierung angibt;
- * eine direkte Bindung an ein Wasserstoffatom oder eine benachbarte A-Gruppierung darstellt; und
- ** eine direkte Bindung zu einer benachbarten A-Gruppierung darstellt.

9. Glycosaminoglycan zur Verwendung gemäß einem der Ansprüche 1 und 3 bis 8 oder Therapeutikum zur Verwendung gemäß einem der Ansprüche 2 bis 8, wobei das Glycosaminoglycan ist:
(a) ein Heparin oder ein Derivat davon oder
(b) Chondroitinsulfat A, Chondroitinsulfat C, Chondroitinsulfat D, Chondroitinsulfat E, Keratansulfat, Heparansulfat oder ein Derivat von einem beliebigen davon.

10. Glycosaminoglycan zur Verwendung gemäß einem der Ansprüche 1 und 3 bis 9 oder Therapeutikum zur Verwendung gemäß einem der Ansprüche 2 bis 9, wobei das Natriumsalz von Heparin oder Heparinsulfat verwendet wird.

11. Glycosaminoglycan zur Verwendung gemäß einem der Ansprüche 1 und 3 bis 10 oder Therapeutikum zur Verwendung gemäß einem der Ansprüche 2 bis 10, wobei das Glycosaminoglycan oder Salz:
(a) Antikoagulanzaktivität beibehält und/oder
(b) keiner Fragmentierung und/oder Depolymerisation unterworfen wurde.

12. Glycosaminoglycan zur Verwendung gemäß einem der Ansprüche 1 und 3 bis 11 oder Therapeutikum zur Verwendung gemäß einem der Ansprüche 2 bis 11, wobei das Verhältnis der Menge von Glycosaminoglycan oder Salz zur der Menge an Therapeutikum, die verabreicht werden, von 500:1 bis 1:500 ist.

13. Glycosaminoglycan oder Therapeutikum zur Verwendung gemäß Anspruch 12, wobei das Verhältnis von 25:1 bis 1:25 ist.

14. Glycosaminoglycan zur Verwendung gemäß einem der Ansprüche 1 und 3 bis 13 oder Therapeutikum zur Verwendung gemäß einem der Ansprüche 2 bis 13, wobei
(a) das Glycosaminoglycan oder Salz und das Therapeutikum zur gleichen Zeit verabreicht werden;
(b) das Glycosaminoglycan oder Salz vor oder nach dem Therapeutikum verabreicht wird und/oder
(c) das Glycosaminoglycan oder Salz und das Therapeutikum als getrennte Medikamente verabreicht werden.

15. Glycosaminoglycan zur Verwendung gemäß einem der Ansprüche 1 und 3 bis 14 oder Therapeutikum zur Verwendung gemäß einem der Ansprüche 2 bis 14, wobei das Therapeutikum aus einem Bronchodilatator, einem Mukolytikum, einem Mukokinetikum, einem anti-inflammatorischen Mittel, einem Proteaseinhibitor, α1-Antitrypsin, einem Mukoregulator, einem anti-pathogenen Mittel, einem Gentherapievektor und einem Antioxidans und einem Steroid ausgewählt ist.

16. Glycosaminoglycan oder Therapeutikum zur Verwendung gemäß Anspruch 15, wobei das anti-pathogene Mittel ein Antibiotikum, ein antivirales Mittel oder ein antifungales Mittel ist.

17. Glycosaminoglycan oder Therapeutikum zur Verwendung gemäß Anspruch 15, wobei der Proteaseinhibitor aus einem Inhibitor von Elastase, einem Cathepsin oder einer Matrixmetalloproteinase (MMP) ausgewählt ist.

18. Glycosaminoglycan zur Verwendung gemäß einem der Ansprüche 1 und 3 bis 17 oder Therapeutikum zur Verwendung gemäß einem der Ansprüche 2 bis 17, wobei das Subjekt ein FEV1 von weniger als 70% des erwarteten Wertes für ein entsprechendes Subjekt, das die Störung nicht hat, hat.

19. Glycosaminoglycan zur Verwendung gemäß einem der Ansprüche 1 und 3 bis 18 oder Therapeutikum zur Verwendung gemäß einem der Ansprüche 2 bis 18, wobei die Atemwegserkrankung oder -Störung durch Mukus-Hypersekretion oder erhöhte Mukus-Viskosität gekennzeichnet ist.

20. Glycosaminoglycan zur Verwendung gemäß einem der Ansprüche 1 und 3 bis 19 oder Therapeutikum zur Verwendung gemäß einem der Ansprüche 2 bis 19, wobei das Medikament ein trockenes Pulver ist.

21. Produkte, umfassend ein Glycosaminoglycan, ausgewählt aus Chondroitinsulfaten A bis E, Heparin, Heparinsulfat, Heparan, Heparansulfat und Keratansulfat, oder ein physiologisch annehmbares Salz eines solchen Glycosaminoglycans und ein Therapeutikum zur gleichzeitigen, getrennten oder sequenziellen Verwendung bei der Behandlung eines menschlichen Subjektes mit einer Atemwegsstörung oder-erkrankung, die erhöhte Level und/oder Viskosität von Mukus oder anderen pulmonalen Sekretionen umfasst, wobei die Erkrankung oder Störung aus chronischer Einschränkung des Luftflusses (CAL), Pneumonie, Sinusitis, Sinuskongestion, zystischer Fibrose, Asthma, Bronchitis, Tracheobronchitis, Bronchiektasie, Tuberkulose, Pilzinfektion, Virusinfektion oder einer Respirationstraktinfektion ausgewählt ist, wobei:
- das Glycosaminoglycan oder Salz und das Therapeutikum durch Inhalation, intranasal und/oder durch Instillation zu verabreichen sind;
- das Glycosaminoglycan oder Salz wie in einem der Ansprüche 1 und 9 bis 12 definiert sind und ein durchschnittliches Molekulargewicht von 12 bis 18 kd haben;
- das Mittel an den Respirationstrakt abzugeben ist;
- der Mukus oder die anderen pulmonalen Sekretionen extrazelluläre genomische DNA enthalten, die die Lungenfunktion gefährdet, und
- das Glycosaminoglycan die Abgabe des Therapeutikums erhöht.

22. Verwendung eines Glycosaminoglycans, ausgewählt aus Chondroitinsulfaten A bis E, Heparin, Heparinsulfat, Heparan, Heparansulfat und Keratansulfat, oder eines physiologisch annehmbaren Salzes eines solchen Glycosaminoglycans für die Herstellung eines Medikaments zur Behandlung eines Säugetiersubjektes mit einer Atemwegsstörung oder-erkrankung, die erhöhte Level und/oder Viskosität von Mukus oder anderen pulmonalen Sekretionen umfasst, wobei die Erkrankung oder Störung aus chronischer Einschränkung des Luftflusses (CAL), Pneumonie, Sinusitis, Sinuskongestion, zystischer Fibrose, Asthma, Bronchitis, Tracheobronchitis, Bronchiektasie, Tuberkulose, Pilzinfektion, Virusinfektion oder einer Respirationstraktinfektion ausgewählt ist, wobei:
- das Subjekt mit einem Therapeutikum behandelt wird;
- das Glycosaminoglycan oder Salz und das Therapeutikum durch Inhalation, intranasal und/oder durch Instillation zu verabreichen sind;
- das Mittel an den Respirationstrakt zu verabreichen ist;
- das Glycosaminoglycan oder Salz davon ein durchschnittliches Molekulargewicht von 12 bis 18 kd hat;
- der Mukus oder die anderen pulmonalen Sekretionen extrazelluläre genomische DNA enthalten, die die Lungenfunktion gefährdet, und
- das Glycosaminoglycan die Abgabe des Therapeutikums erhöht.

23. Verwendung eines Therapeutikums für die Herstellung eines Medikaments zur Behandlung eines Säugetiersubjektes mit einer Atemwegsstörung oder -erkrankung, die erhöhte Level und/oder Viskosität von Mukus oder anderen pulmonalen Sekretionen umfasst, wobei die Erkrankung oder Störung aus chronischer Einschränkung des Luftflusses (CAL), Pneumonie, Sinusitis, Sinuskongestion, zystischer Fibrose, Asthma, Bronchitis, Tracheobronchitis, Bronchiektasie, Tuberkulose, Pilzinfektion, Virusinfektion oder einer Respirationstraktinfektion ausgewählt ist, wobei:
- dem Subjekt auch ein Glycosaminoglycan, ausgewählt aus Chondroitinsulfaten A bis E, Heparin, Heparinsulfat, Heparan, Heparansulfat und Keratansulfat, oder ein physiologisch annehmbares Salz eines solchen Glycosaminoglycans verabreicht wird;
- das Glycosaminoglycan oder Salz und das Therapeutikum durch Inhalation, intranasal und/oder durch Instillation zu verabreichen sind;
- das Mittel an den Respirationstrakt abzugeben ist;
- das Glycosaminoglycan oder Salz davon ein durchschnittliches Molekulargewicht von 12 bis 18 kd hat;
- der Mukus oder die anderen pulmonalen Sekretionen extrazelluläre genomische DNA enthalten, welche die Lungenfunktion gefährdet, und
- das Glycosaminoglycan die Abgabe des Therapeutikums erhöht.

24. Produkte zur Verwendung gemäß Anspruch 21 oder Verwendung gemäß Anspruch 22 oder 23, wobei:
- das Therapeutikum wie in einem der Ansprüche 12 bis 14 definiert ist und/oder
- das Verhältnis von Glycosaminoglycan oder Salz zu dem Therapeutikum wie in Anspruch 12 oder 13 definiert ist.

25. Produkte zur Verwendung gemäß Anspruch 21 oder 24 oder Verwendung gemäß einem der Ansprüche 22 bis 24, wobei das Subjekt wie in einem der Ansprüche 3 bis 7 definiert ist.

## Revendications

1. Glycosaminoglycane choisi parmi les chondroïtine-sulfates A à E, l'héparine, l'héparine-sulfate, l'héparane, l'héparane-sulfate et le kératane-sulfate, ou sel physiologiquement admissible d'un tel glycosaminoglycane, pour utilisation dans un procédé de traitement d'un sujet mammifère présentant un trouble ou une maladie respiratoire qui comporte une augmentation des niveaux et/ou de la viscosité du mucus ou d'autres sécrétions pulmonaires, lequel trouble ou laquelle maladie est choisi(e) parmi les suivants : limitation chronique du débit aérien (LCDA), pneumonie, sinusite, congestion des sinus, mucoviscidose, asthme, bronchite, trachéo-bronchite, bronchiectasie, tuberculose, infection fongique, infection virale et infection des voies respiratoires, étant entendu que :
- le sujet se trouve sous traitement par un agent thérapeutique ;
- le glycosaminoglycane ou son sel et l'agent thérapeutique sont administrés par inhalation, par voie intranasale et/ou par instillation ;
- l'agent doit être délivré dans les voies respiratoires ;
- ledit glycosaminoglycane ou son sel ont une masse molaire moyenne de 12 à 18 kDa ;
- le mucus ou les autres sécrétions pulmonaires contiennent de l'ADN génomique extracellulaire qui compromet les fonctions pulmonaires ;
- et le glycosaminoglycane favorise la délivrance de l'agent thérapeutique.

2. Agent thérapeutique pour utilisation dans un procédé de traitement d'un sujet mammifère présentant un trouble ou une maladie respiratoire qui comporte une augmentation des niveaux et/ou de la viscosité du mucus ou d'autres sécrétions pulmonaires, lequel trouble ou laquelle maladie est choisi(e) parmi les suivants : limitation chronique du débit aérien (LCDA), pneumonie, sinusite, congestion des sinus, mucoviscidose, asthme, bronchite, trachéo-bronchite, bronchiectasie, tuberculose, infection fongique, infection virale et infection des voies respiratoires, étant entendu que :
- on administre aussi au sujet un glycosaminoglycane choisi parmi les chondroïtine-sulfates A à E, l'héparine, l'héparine-sulfate, l'héparane, l'héparane-sulfate et le kératane-sulfate, ou un sel physiologiquement admissible d'un tel glycosaminoglycane ;
- le glycosaminoglycane ou son sel et l'agent thérapeutique sont administrés par inhalation, par voie intranasale et/ou par instillation ;
- l'agent doit être délivré dans les voies respiratoires ;
- ledit glycosaminoglycane ou son sel a une masse molaire moyenne de 12 à 18 kDa ;
- le mucus ou les autres sécrétions pulmonaires contiennent de l'ADN génomique extracellulaire qui compromet les fonctions pulmonaires ;
- et le glycosaminoglycane favorise la délivrance de l'agent thérapeutique.

3. Glycosaminoglycane pour utilisation conforme à la revendication 1, ou agent thérapeutique pour utilisation conforme à la revendication 2, étant entendu que le sujet présente une infection des voies respiratoires.

4. Glycosaminoglycane ou agent thérapeutique pour utilisation conforme à la revendication 3, étant entendu que l'infection est une infection bactérienne ou virale.

5. Glycosaminoglycane ou agent thérapeutique pour utilisation conforme à la revendication 4, étant entendu que l'infection virale est une grippe ou un rhume.

6. Glycosaminoglycane pour utilisation conforme à l'une des revendications 1 et 3 à 5, ou agent thérapeutique pour utilisation conforme à l'une des revendications 2 à 5, étant entendu que le sujet présente une limitation chronique du débit aérien (LCDA).

7. Glycosaminoglycane pour utilisation conforme à l'une des revendications 1 et 3 à 6, ou agent thérapeutique pour utilisation conforme à l'une des revendications 2 à 6, étant entendu que le sujet est un humain.

8. Glycosaminoglycane pour utilisation conforme à l'une des revendications 1 et 3 à 7, ou agent thérapeutique pour utilisation conforme à l'une des revendications 2 à 7, étant entendu que le glycosaminoglycane, ou son sel physiologiquement admissible, comprend des motifs répétés de type disaccharide, de formule générale (1) :
-[A-B]- (1)
dans lesquels motifs
- chaque symbole A représente une entité, identique ou différente, qui est un fragment de formule (i) ou (ii) : 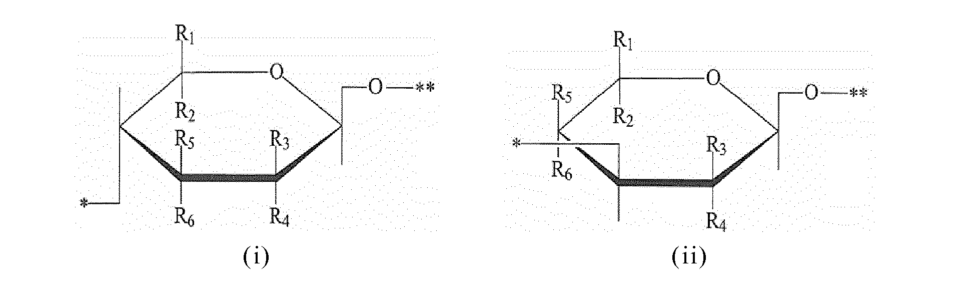 dans lesquelles formules
- l'un des symboles R₁ et R₂ représente un atome d'hydrogène et l'autre représente un groupe symbolisé par -CO₂H, -SO₃H ou -CH₂OR où R représente un atome d'hydrogène ou un groupe -SO₃H,
- l'un des symboles R₃ et R₄ représente un atome d'hydrogène et l'autre représente un groupe symbolisé par -OR où R représente un atome d'hydrogène ou un groupe -SO₃H,
- l'un des symboles R₅ et R₆ représente un atome d'hydrogène et l'autre représente un groupe -OH,
- le symbole * représente une liaison directe vers un atome d'hydrogène ou un fragment B adjacent,
- et le symbole ** représente une liaison directe vers un fragment B adjacent ;
- et chaque symbole B représente une entité, identique ou différente, qui est un fragment de formule (iii) ou (iv) : 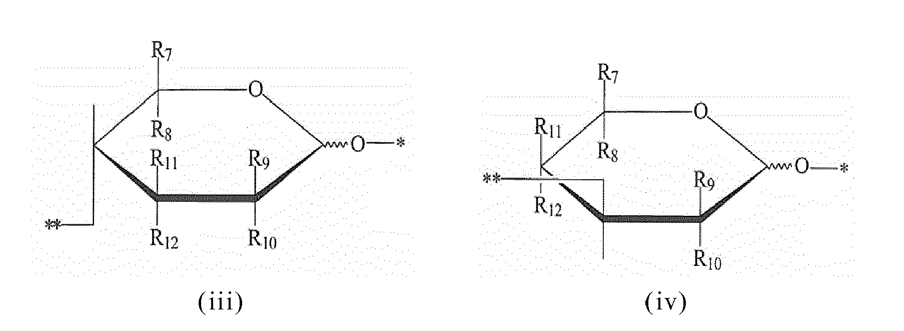 dans lesquelles formules
- l'un des symboles R₇ et R₈ représente un atome d'hydrogène et l'autre représente un groupe -CH₂OH ou -CH₂OSO₃H,
- l'un des symboles R₉ et R₁₀ représente un atome d'hydrogène et l'autre représente un groupe -NHAc, -NH₂ ou -NHSO₃H,
- l'un des symboles R₁₁ et R₁₂ représente un atome d'hydrogène et l'autre représente un groupe -OH ou -OSO₃H,
- l'élément 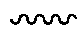 indique une liaison qui se trouve dans l'une ou l'autre orientation stéréochimique,
- le symbole * représente une liaison directe vers un atome d'hydrogène ou un fragment A adjacent, et
- et le symbole ** représente une liaison directe vers un fragment A adjacent.

9. Glycosaminoglycane pour utilisation conforme à l'une des revendications 1 et 3 à 8, ou agent thérapeutique pour utilisation conforme à l'une des revendications 2 à 8, étant entendu que le glycosaminoglycane
a) est de l'héparine ou un dérivé de celle-ci,
b) ou est du chondroïtine-sulfate A, du chondroïtine-sulfate C, du chondroïtine-sulfate D, du chondroïtine-sulfate E, du kératane-sulfate ou de l'héparane-sulfate, ou un dérivé de n'importe lequel d'entre ceux-ci.

10. Glycosaminoglycane pour utilisation conforme à l'une des revendications 1 et 3 à 9, ou agent thérapeutique pour utilisation conforme à l'une des revendications 2 à 9, étant entendu que l'on utilise un sel de sodium d'héparine ou d'héparine-sulfate.

11. Glycosaminoglycane pour utilisation conforme à l'une des revendications 1 et 3 à 10, ou agent thérapeutique pour utilisation conforme à l'une des revendications 2 à 10, étant entendu que le glycosaminoglycane ou son sel
a) conserve une activité d'anti-coagulant,
b) et/ou n'a subi ni fragmentation, ni dépolymérisation.

12. Glycosaminoglycane pour utilisation conforme à l'une des revendications 1 et 3 à 11, ou agent thérapeutique pour utilisation conforme à l'une des revendications 2 à 11, étant entendu que le rapport de la quantité de glycosaminoglycane ou de son sel à la quantité de médicament thérapeutique administrée vaut de 500/1 à 1/500.

13. Glycosaminoglycane ou agent thérapeutique pour utilisation conforme à la revendication 12, étant entendu que le rapport vaut de 25/1 à 1/25.

14. Glycosaminoglycane pour utilisation conforme à l'une des revendications 1 et 3 à 13, ou agent thérapeutique pour utilisation conforme à l'une des revendications 2 à 13, étant entendu que
a) le glycosaminoglycane ou son sel et l'agent thérapeutique sont administrés en même temps,
b) ou le glycosaminoglycane ou son sel est administré avant ou après l'agent thérapeutique,
c) et/ou le glycosaminoglycane ou son sel et l'agent thérapeutique sont administrés sous forme de médicaments séparés.

15. Glycosaminoglycane pour utilisation conforme à l'une des revendications 1 et 3 à 14, ou agent thérapeutique pour utilisation conforme à l'une des revendications 2 à 14, étant entendu que l'agent thérapeutique est choisi parmi un bronchodilatateur, un mucolytique, un mucocinétique, un anti-inflammatoire, un inhibiteur de protéase, l'alpha-1-antitrypsine, un mucorégulateur, un agent anti-pathogène, un vecteur de thérapie génique, un anti-oxydant et un stéroïde.

16. Glycosaminoglycane ou agent thérapeutique pour utilisation conforme à la revendication 15, étant entendu que l'agent anti-pathogène est un antibiotique, un agent antiviral ou un agent antifongique.

17. Glycosaminoglycane ou agent thérapeutique pour utilisation conforme à la revendication 15, étant entendu que l'inhibiteur de protéase est choisi parmi des inhibiteurs d'élastase, d'une cathepsine ou d'une métallo-protéinase matricielle (MMP).

18. Glycosaminoglycane pour utilisation conforme à l'une des revendications 1 et 3 à 17, ou agent thérapeutique pour utilisation conforme à l'une des revendications 2 à 17, étant entendu que le sujet présente un volume VEF1 inférieur à 75 % de la valeur attendue dans le cas d'un sujet équivalent qui n'est pas atteint du trouble.

19. Glycosaminoglycane pour utilisation conforme à l'une des revendications 1 et 3 à 18, ou agent thérapeutique pour utilisation conforme à l'une des revendications 2 à 18, étant entendu que le trouble ou la maladie respiratoire est **caractérisé par** une hypersécrétion de mucus ou par un mucus fortement visqueux.

20. Glycosaminoglycane pour utilisation conforme à l'une des revendications 1 et 3 à 19, ou agent thérapeutique pour utilisation conforme à l'une des revendications 2 à 19, étant entendu que le médicament est une poudre sèche.

21. Produits comprenant un glycosaminoglycane choisi parmi les chondroïtine-sulfates A à E, l'héparine, l'héparine-sulfate, l'héparane, l'héparane-sulfate et le kératane-sulfate, ou un sel physiologiquement admissible d'un tel glycosaminoglycane, et un agent thérapeutique, pour utilisation en mode simultané, séparé ou consécutif dans le traitement d'un sujet humain présentant un trouble ou une maladie respiratoire qui comporte une augmentation des niveaux et/ou de la viscosité du mucus ou d'autres sécrétions pulmonaires, lequel trouble ou laquelle maladie est choisi(e) parmi les suivants : limitation chronique du débit aérien (LCDA), pneumonie, sinusite, congestion des sinus, mucoviscidose, asthme, bronchite, trachéo-bronchite, bronchiectasie, tuberculose, infection fongique, infection virale et infection des voies respiratoires, dans lesquels produits :
- le glycosaminoglycane ou son sel et l'agent thérapeutique doivent être administrés par inhalation, par voie intranasale et/ou par instillation ;
- le glycosaminoglycane ou son sel sont tels que définis dans l'une des revendications 1 et 9 à 12, et ont une masse molaire moyenne de 12 à 18 kDa ;
- l'agent doit être délivré dans les voies respiratoires ;
- le mucus ou les autres sécrétions pulmonaires contiennent de l'ADN génomique extracellulaire qui compromet les fonctions pulmonaires ;
- et le glycosaminoglycane favorise la délivrance de l'agent thérapeutique.

22. Utilisation d'un glycosaminoglycane choisi parmi les chondroïtine-sulfates A à E, l'héparine, l'héparine-sulfate, l'héparane, l'héparane-sulfate et le kératane-sulfate, ou d'un sel physiologiquement admissible d'un tel glycosaminoglycane, pour la fabrication d'un médicament conçu pour le traitement d'un sujet mammifère présentant un trouble ou une maladie respiratoire qui comporte une augmentation des niveaux et/ou de la viscosité du mucus ou d'autres sécrétions pulmonaires, lequel trouble ou laquelle maladie est choisi(e) parmi les suivants : limitation chronique du débit aérien (LCDA), pneumonie, sinusite, congestion des sinus, mucoviscidose, asthme, bronchite, trachéo-bronchite, bronchiectasie, tuberculose, infection fongique, infection virale et infection des voies respiratoires, étant entendu que :
- le sujet se trouve sous traitement par un agent thérapeutique ;
- le glycosaminoglycane ou son sel et l'agent thérapeutique doivent être administrés par inhalation, par voie intranasale et/ou par instillation ;
- l'agent doit être délivré dans les voies respiratoires ;
- ledit glycosaminoglycane ou son sel ont une masse molaire moyenne de 12 à 18 kDa ;
- le mucus ou les autres sécrétions pulmonaires contiennent de l'ADN génomique extracellulaire qui compromet les fonctions pulmonaires ;
- et le glycosaminoglycane favorise la délivrance de l'agent thérapeutique.

23. Utilisation d'un agent thérapeutique pour la fabrication d'un médicament conçu pour le traitement d'un sujet mammifère présentant un trouble ou une maladie respiratoire qui comporte une augmentation des niveaux et/ou de la viscosité du mucus ou d'autres sécrétions pulmonaires, lequel trouble ou laquelle maladie est choisi(e) parmi les suivants : limitation chronique du débit aérien (LCDA), pneumonie, sinusite, congestion des sinus, mucoviscidose, asthme, bronchite, trachéo-bronchite, bronchiectasie, tuberculose, infection fongique, infection virale et infection des voies respiratoires, étant entendu que :
- on administre aussi au sujet un glycosaminoglycane choisi parmi les chondroïtine-sulfates A à E, l'héparine, l'héparine-sulfate, l'hépa-rane, l'héparane-sulfate et le kératane-sulfate, ou un sel physiologiquement admissible d'un tel glycosaminoglycane ;
- le glycosaminoglycane ou son sel et l'agent thérapeutique doivent être administrés par inhalation, par voie intranasale et/ou par instillation ;
- l'agent doit être délivré dans les voies respiratoires ;
- ledit glycosaminoglycane ou son sel ont une masse molaire moyenne de 12 à 18 kDa ;
- le mucus ou les autres sécrétions pulmonaires contiennent de l'ADN génomique extracellulaire qui compromet les fonctions pulmonaires ;
- et le glycosaminoglycane favorise la délivrance de l'agent thérapeutique.

24. Produits pour utilisation conformes à la revendication 21, ou utilisation conforme à la revendication 22 ou 23, étant entendu que :
- l'agent thérapeutique est tel que défini dans l'une des revendications 12 à 14 ;
- et/ou le rapport du glycosaminoglycane ou de son sel à l'agent thérapeutique est tel que défini dans la revendication 12 ou 13.

25. Produits pour utilisation conformes à la revendication 21 ou 24, ou utilisation conforme à l'une des revendications 22 à 24, étant entendu que le sujet est tel que défini dans l'une des revendications 3 à 7.
